# EUROPÄISCHE PATENTSCHRIFT

(11) **EP 0 869 948 B1**
(45) Veröffentlichungstag und Bekanntmachung des Hinweises auf die Patenterteilung: **13.06.2001**
(21) Anmeldenummer: 96904086.4
(22) Anmeldetag: 01.03.1996
(51) Int. Cl.: C07D 249/12, A01N 47/38, C07D 401/12, C07D 403/12, C07D 405/12, C07D 409/12, C07D 417/12

(54) **HERBIZIDE ODER FUNGIZIDE SULFONYLAMINOCARBONYLTRIAZOLINONE MIT HALOGENALK(EN)YLTHIO-SUBSTITUENTEN**
HERBICIDAL OR FUNGICIDAL SULPHONYLAMINOCARBONYLTRIAZOLINONES WITH HALOGENATED ALK(EN)YLTHIO SUBSTITUENTS
SULFONYLAMINOCARBONYLTRIAZOLINONES HERBICIDES OU FONGICIDES A SUBSTITUANTS ALC(EN)YLTHIO HALOGENES

(30) Priorität: 08.03.1995 DE 19508119
(43) Veröffentlichungstag der Anmeldung: 14.10.1998
(73) Patentinhaber: BAYER AG, 51368 Leverkusen (DE)
(72) Erfinder: MÜLLER, Klaus-Helmut, D-40593 Düsseldorf (DE); KIRSTEN, Rolf, D-40789 Monheim (DE); GESING, Ernst, Rudolf, F., D-40699 Erkrath (DE); KLUTH, Joachim, D-40764 Langenfeld (DE); FINDEISEN, Kurt, D-51375 Leverkusen (DE); JANSEN, Johannes, R., D-40789 Monheim (DE); KÖNIG, Klaus, D-51519 Odenthal (DE); RIEBEL, Hans-Jochem, D-42113 Wuppertal (DE); BIELEFELDT, Dietmar, D-40883 Ratingen (DE); DOLLINGER, Markus, D-51381 Leverkusen (DE); SANTEL, Hans-Joachim, D-51371 Leverkusen (DE); STENZEL, Klaus, D-40595 Düsseldorf (DE)
(86) Internationale Anmeldenummer: EP9600834
(87) Internationale Veröffentlichungsnummer: WO9627591

(56) Entgegenhaltungen:
- EP-A- 0 341 489
- DE-A- 2 412 564
- DE-A- 3 936 623
- US-A- 4 098 896

## Beschreibung

Die Erfindung betrifft neue Sulfonylaminocarbonyltriazolinone mit Halogenalkylthio-Substituenten, mehrere Verfahren und neue Zwischenprodukte zu ihrer Herstellung sowie ihre Verwendung als Herbizide und Fungizide.

Es ist bereits bekannt, daß bestimmte Sulfonylaminocarbonyltriazolinone herbizide Eigenschaften aufweisen (vgl. EP-A 34148, EP-A 422469, EP-A 425948, EP-A 431291, EP-A 507171). Die Wirkung dieser Verbindungen ist jedoch nicht in allen Belangen zufriedenstellend.

Es wurden nun die neuen Sulfonylaminocarbonyltriazolinone mit Halogenalkylthio-Substituenten der allgemeinen Formel (I), 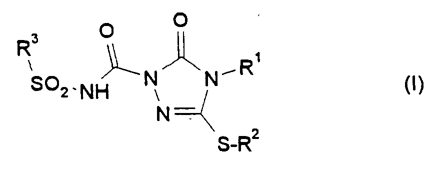 in welcher
- R¹: für Wasserstoff, Hydroxy, Amino, Alkylidenamino oder für einen jeweils gegebenenfalls substituierten Rest aus der Reihe Alkyl, Alkenyl, Alkinyl, Alkylamino, Dialkylamino, Alkanoylamino, Cycloalkyl, Cycloalkylalkyl, Cycloalkylamino, Aryl, Arylalkyl steht,
- R²: für jeweils durch Halogen substituiertes Alkyl oder Alkenyl steht und
- R³: für einen jeweils gegebenenfalls substituierten Rest aus der Reihe Alkyl, Aralkyl, Aryl, Heteroaryl steht,
sowie Salze von Verbindungen der Formel (I) gefunden.

Man erhält die neuen Sulfonylaminocarbonyltriazolinone mit Halogenalkylthio-Substituenten der allgemeinen Formel (I), wenn man
(a) Triazolinone der allgemeinen Formel (II) 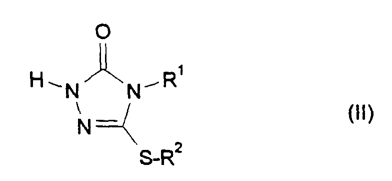 in welcher
   - R¹ und R²: die oben angegebene Bedeutung haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(b) Triazolinon-Derivate der allgemeinen Formel (IV) 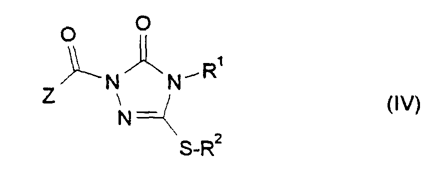 in welcher
   - R¹ und R²: die oben angegebene Bedeutung haben und
   - Z: für Halogen, Alkoxy, Aralkoxy oder Aryloxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   oder wenn man
(c) Triazolinone der allgemeinen Formel (II) 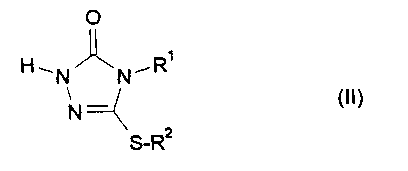 in welcher
   - R¹ und R²: die oben angegebene Bedeutung haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Halogen, Alkoxy, Aralkoxy oder Aryloxy steht,
   gegebenenfalls in Gegenwart eines Säureakzeptors und gegebenenfalls in Gegenwart eines Verdünnungsmittels umsetzt,
   oder wenn man
(d) Triazolinone der allgemeinen Formel (II) 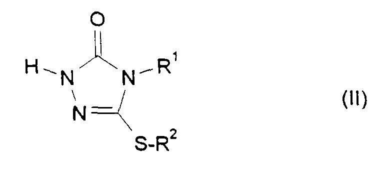 in welcher
   - R¹ und R²: die oben angegebene Bedeutung haben,
   mit Sulfonsäurehalogeniden der allgemeinen Formel (VII)

   R³-SO₂-X (VII)

   in welcher
   - R³: die oben angegebene Bedeutung hat und
   - X: für Halogen steht,
   und Metallcyanaten der allgemeinen Formel (VIII)

   MOCN (VIII)

   in welcher
   - M: für ein Alkalimetall oder Erdalkalimetall-äquivalent steht,
   gegebenenfalls in Gegenwart eines Reaktionshilfsmittels und gegebenenfalls in Gegenwart eines Verdünnungsmittels umsetzt,
   und gegebenenfalls die nach Verfahren (a), (b), (c) oder (d) erhaltenen Verbindungen der Formel (I) nach üblichen Methoden in Salze überführt.

Die neuen Sulfonylaminocarbonyltriazolinone mit Halogenalkylthio-Substituenten der allgemeinen Formel (I) zeichnen sich durch starke herbizide und/oder fungizide Wirksamkeit aus.

Gegenstand der Erfindung sind vorzugsweise Verbindungen der Formel (I), in welcher
- R¹: für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor und/oder Chlor substituiertes C₁-C₆-Alkylamino, Di-(C₁-C₄-alkyl)-amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl. Trifluormethyl, C₁-C₄-Alkoxy und/oder C₁-C₄-Alkoxy-carbonyl substituiertes Phenyl oder Phenyl-C₁-C₄-alkyl steht,
- R²: für jeweils durch Fluor, Chlor und/oder Brom substituiertes C₁-C₆-Alkyl oder C₂-C₆-Alkenyl steht und
- R³: für die Gruppierung 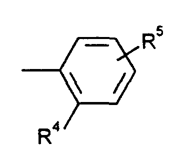 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxy-carbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkyl-sulfinyl oder C₁-C₄-Alkylsulfonyl substituiert ist), für C₂-C₆-Alkenyl-oxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy- carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
R⁴ und/oder R⁵ weiterhin für Phenyl oder Phenoxy, für C₁-C₄-Alkyl-carbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkylamino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonylamino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht, weiterhin
R³ für den Rest 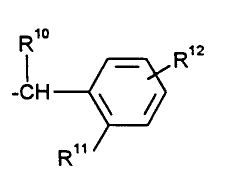 steht,
worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxycarbonyl, Dimethylaminocarbonyl, C₁-C₄-Alkylsulfonyl oder Di-(C₁-C₄-alkyl)-aminosulfonyl stehen; weiterhin

- R³: für den Rest 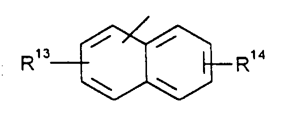 steht,
worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen; weiterhin
- R³: für den Rest 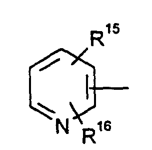 steht,
worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), für C₁-C₄-Alkylthio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen; weiterhin
- R³: für den Rest 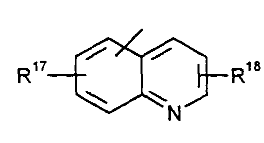 steht,
worin
R¹⁷ und R¹⁸ gleich oder verschieden sind und für Wasserstoff, Fluor, Chlor, Brom, C₁-C₄-Alkyl (welches gegebenenfalls durch Fluor und/-oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen; weiterhin

- R³: für den Rest 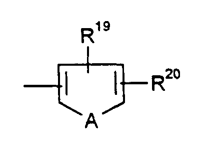 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-aminosulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxycarbonyl oder Di-(C₁-C₄-alkyl)-aminocarbonyl steht; weiterhin
R³ für den Rest 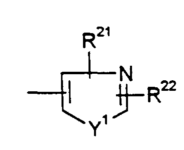 steht,
worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
- R³: für den Rest 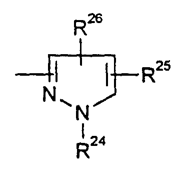 steht,
worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher n, R¹, R² und R³ die oben vorzugsweise angegebenen Bedeutungen haben.

Gegenstand der Erfindung sind insbesondere Verbindungen der Formel (I), in welcher
- R¹: für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für Methylamino, Ethylamino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
- R²: für jeweils durch Fluor und/oder Chlor substituiertes Methyl, Ethyl, n- oder i-Propyl, n-, i-, s- oder t-Butyl, Propenyl oder Butenyl steht und
- R³: für den Rest 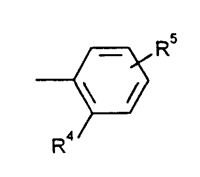 steht,
worin
R⁴ für Fluor, Chlor, Brom, Methyl, Ethyl, n- oder i-Propyl, Trifluormethyl, Methoxy, Ethoxy, n- oder i-Propoxy, n- oder i-Butoxy, Difluormethoxy, Trifluormethoxy, 2-Chlor-ethoxy, 2,2,2-Trifluor-ethoxy, 1,1,2,2-Tetrafluor-ethoxy, 1,1,2,2,2-Pentafluor-ethoxy, 2-Methoxy-ethoxy, Methylthio, Ethylthio, n- oder i-Propylthio, n- oder i-Butylthio, 2-Fluor-ethylthio, Allyloxy, Propargyloxy, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n- oder i-Propoxycarbonyl, steht und
R⁵ für Wasserstoff, Methyl, Ethyl, Fluor, Chlor oder Brom steht; weiterhin
R³ für den Rest 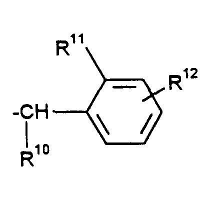 steht,
worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
- R³: für den Rest 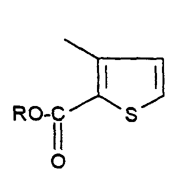 steht,
worin
R für Methyl, Ethyl, n- oder i-Propyl steht, oder
- R³: für den Rest 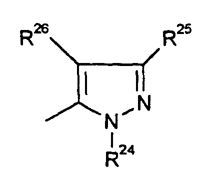 steht,
worin
R²⁴ für Methyl, Ethyl, n- oder i-Propyl, Phenyl oder Pyridyl steht,
R²⁵ für Wasserstoff, Fluor, Chlor oder Brom steht und
R²⁶ für Fluor, Chlor, Brom, Methoxycarbonyl oder Ethoxycarbonyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man beispielsweise 2-Trifluormethoxy-phenylsulfonyl-isocyanat und 4-Ethyl-5-difluormethylthio-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

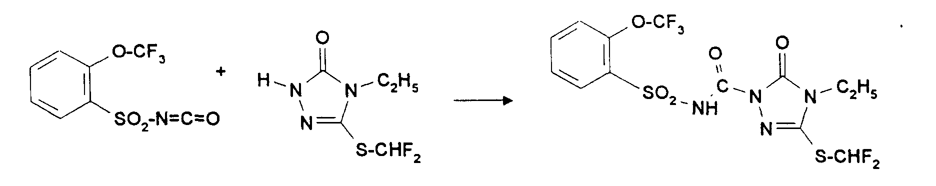

Verwendet man beispielsweise 2-Ethylthio-benzolsulfonamid und 2-Chlorcarbonyl-4-cyclopropyl-5-(2,2-difluor-ethylthio)-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

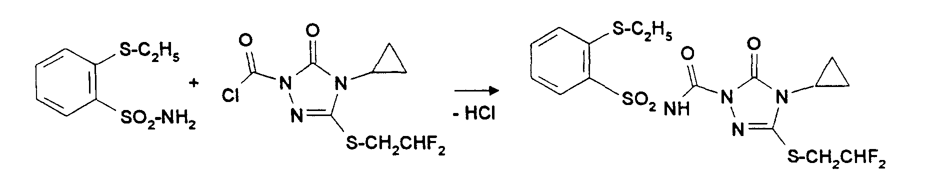

Verwendet man beispielsweise N-Methoxycarbonyl-2-methoxy-benzolsulfonamid und 5-(2-Chlor-ethylthio)-4-methyl-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

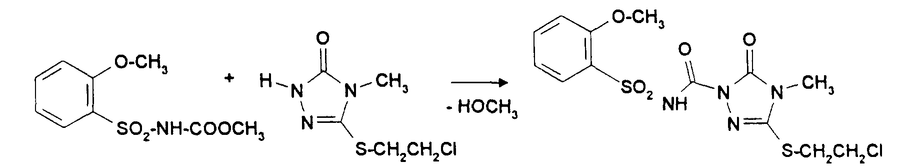

Verwendet man beispielsweise 2-Chlor-6-methyl-benzolsulfonsäurechlorid, 4-Allyl-5-(2,3,3-trifluor-propylthio)-2,4-dihydro-3H-1,2,4-triazol-3-on und Natriumcyanat als Ausgangsstoffe, so kann der Reaktionsablauf beim erfindungsgemäßen Verfahren (d) durch das folgende Formelschema skizziert werden:

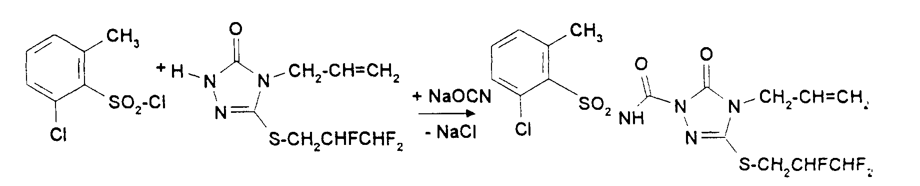

Die bei den erfindungsgemäßen Verfahren (a), (c) und (d) zur Herstellung von Verbindungen der Formel (I) als Ausgangsstoffe zu verwendenden Triazolinone sind durch die Formel (II) allgemein definiert.

In der Formel (II) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Triazolinone der allgemeinen Formel (II) sind noch nicht aus der Literatur bekannt; sie sind als neue Stoffe auch Gegenstand der vorliegenden Anmeldung.

Man erhält die neuen Triazolinone der Formel (II), wenn man Mercaptotriazolinone der allgemeinen Formel (IX) 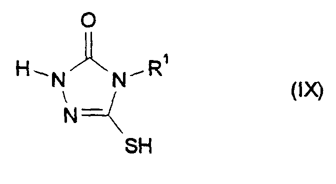 in welcher
- R¹: die oben angegebene Bedeutung hat,
- und/oder die entsprechenden hierzu isomeren Thiourazole und/oder entsprechende Metallsalze, insbesondere Natrium- oder Kaliumsalze -
mit Alkylierungmitteln der allgemeinen Formel (X)

X¹-R² (X)

in welcher
- R²: die oben angegebene Bedeutung hat und
- X¹: für Halogen, vorzugsweise Chlor, Brom oder Iod steht,
gegebenenfalls in Gegenwart eines Säureakzeptors, wie z.B. Natriumhydroxid, und in Gegenwart eines Verdünnungsmittels, wie z.B. Dioxan, Methanol, Ethanol, n-oder i-Propanol und/oder Wasser, bei Temperaturen zwischen 0°C und 100°C umsetzt (vgl. die Herstellungsbeispiele).

Die Vorprodukte der Formel (IX) sind bekannt und/oder können nach an sich bekannten Verfahren hergestellt werden (vgl. EP-A 431291; DE-A 2250572; J. Hetero-cycl. Chem. 15 (1978), 377-384).

Die Vorprodukte der Formel (X) sind ebenfalls bekannt und/oder können nach an sich bekannten Verfahren hergestellt werden (vgl. J. Fluorine Chem. 13 (1979), 325; loc. cit. 20 (1982), 637; loc. cit. 21 (1982), 253; loc. cit. 28 (1985), 291); J. Chem. Soc., Perkin II 1975, 1841).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In der Formel (III) hat R³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ angegeben wurde.

Die Ausgangsstoffe der Formel (III) sind bekannt und/oder können nach an sich bekannten Verfahren hergestellt werden (vgL US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (b) zur Herstellung der Verbindungen der allgemeinen Formel (I) als Ausgangsstoffe zu verwendenden Triazolinon-Derivate sind durch die Formel (IV) allgemein definiert. In der Formel (IV) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; Z steht vorzugsweise für Fluor, Chlor, Brom, Methoxy, Ethoxy, Benzyloxy, Phenoxy, Halogen- oder Nitro-phenoxy, insbesondere für Methoxy, Phenoxy oder 4-Nitro-phenoxy.

Die Ausgangsstoffe der Formel (IV) sind noch nicht aus der Literatur bekannt; sie sind als neue Stoffe ebenfalls Gegenstand der vorliegenden Anmeldung.

Man erhält die neuen Verbindungen der Formel (IV), wenn man Triazolinone der allgemeinen Formel (II) 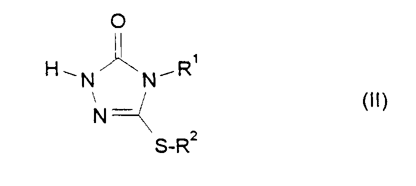 in welcher
- R¹ und R²: die oben angegebene Bedeutung haben,
mit Kohlensäurederivaten der allgemeinen Formel (XIII)

Z-CO-Z¹ (XIII)

in welcher
- Z: die oben angegebene Bedeutung hat und
- Z¹: für Halogen, Alkoxy, Aralkoxy oder Aryloxy steht,
gegebenenfalls in Gegenwart eines Säureakzeptors, wie z.B. Natrium- oder Kaliumhydrid, Natrium- oder Kaliumhydroxid, Natrium- oder Kalium-t-butylat, und gegebenenfalls in Gegenwart eines Verdünnungsmittels, wie z.B. Tetrahydrofuran oder Dimethoxyethan, oder in einem Zweiphasensystem aus Wasser und einem organischen Lösungsmittel, wie z.B. Methylenchlorid oder Chloroform, bei Temperaturen zwischen 0°C und 100°C umsetzt.

Die beim erfindungsgemäßen Verfahren (b) zur Herstellung der Verbindungen der allgemeinen Formel (I) weiter als Ausgangsstoffe zu verwendenden Sulfonsäureamide sind durch die Formel (V) allgemein definiert. In der Formel (V) hat R³ vorzugsweise bzw, insbesondere diejenige Bedeutung, die bereits oben im Zusammenhang mit der Beschreibung der Verbindungen der Formel (I) als bevorzugt bzw. als insbesondere bevorzugt für R³ angegeben wurde.

Die Ausgangsstoffe der Formel (V) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (c) zur Herstellung der Verbindungen der Formel (I) als Ausgangsstoffe zu verwendenden Sulfonsäureamid-Derivate sind durch die Formel (VI) allgemein definiert. In der Formel (VI) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; Z steht vorzugsweise für Fluor, Chlor, Brom, Methoxy, Ethoxy, Benzyloxy oder Phenoxy, insbesondere für Methoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung der Verbindungen der Formel (I) als Ausgangsstoffe zu verwendenden Sulfonsäurehalogenide sind durch die Formel (VII) allgemein definiert. In der Formel (VII) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; X steht vorzugsweise für Fluor, Chlor oder Brom, insbesondere für Chlor.

Die Ausgangsstoffe der Formel (VII) sind bekannt und/oder können nach an sich bekannten Verfahren hergestellt werden.

Die erfindungsgemäßen Verfahren (a), (b), (c) und (d) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-,

Methyl-isopropyl- und Methyl-isobutyl-keton; Ester wie Essigssäuremethylester und - ethylester; Nitrile wie z.B. Acetonitril und Propionitril; Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c) und (d) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c) und (d) in einem größeren Bereich variiert werden. Im allgemeinen arbeitet man bei Temperaturen zwischen -20°C und +100°C, vorzugsweise bei Temperaturen zwischen 0°C und +80°C.

Die erfindungsgemäßen Verfahren (a), (b), (c) und (d) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c) und (d) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.
Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen sowie auf Weideflächen eingesetzt werden. Ferner können die Verbindungen zur Totalunkrautbekämpfung wie auch zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die erfindungsgemäßen Wirkstoffe weisen zusätzlich auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Verbindungen der Formel (I) eignen sich insbesondere zur protektiven Behandlung von Kernobst, wie z.B. Äpfeln, gegen den Apfelmehltauerreger (Podosphaera leucotricha) und in gewissem Umfang auch zum Einsatz im Reis gegen Pyricularia oryzae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Ouarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether. wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron -ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenflache. vorzugsweise zwischen 5 g und 2 kg pro ha.

Die Herstellung und die Verwendung der erfindungsgemäßen Wirkstoffe geht aus den nachfolgenden Beispielen hervor.

### Herstellungsbeispiele:

### Beispiel 1

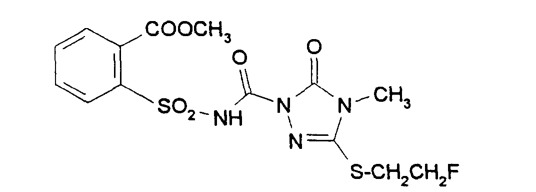

### (Verfahren (a))

1,8 g (10 mMol) 5-(2-Fluor-ethylthio)-4-methyl-2,4-dihydro-3H-1,2,4-triazol-3-on werden in 50 ml Acetonitril gelöst und mit 2,65 g (11 mMol) 2-Methoxycarbonylphenylsulfonylisocyanat versetzt. Nach 12 Stunden Rühren bei 20°C wird im Vakuum eingeengt, der Rückstand mit Isopropanol/Petrolether (1:1) verrührt und zur Kristallisation gebracht.

Man erhält 3,8 g (91% der Theorie) 5-(2-Fluor-ethylthio)-4-methyl-2-(2-methoxy-carbonyl-phenylsulfonyl-aminocarbonyl)-2,4-dihydro-3H-1,2,4-triazol-3-on vom Schmelzpunkt 132°C.

Analog Beispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

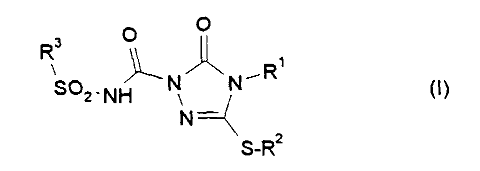

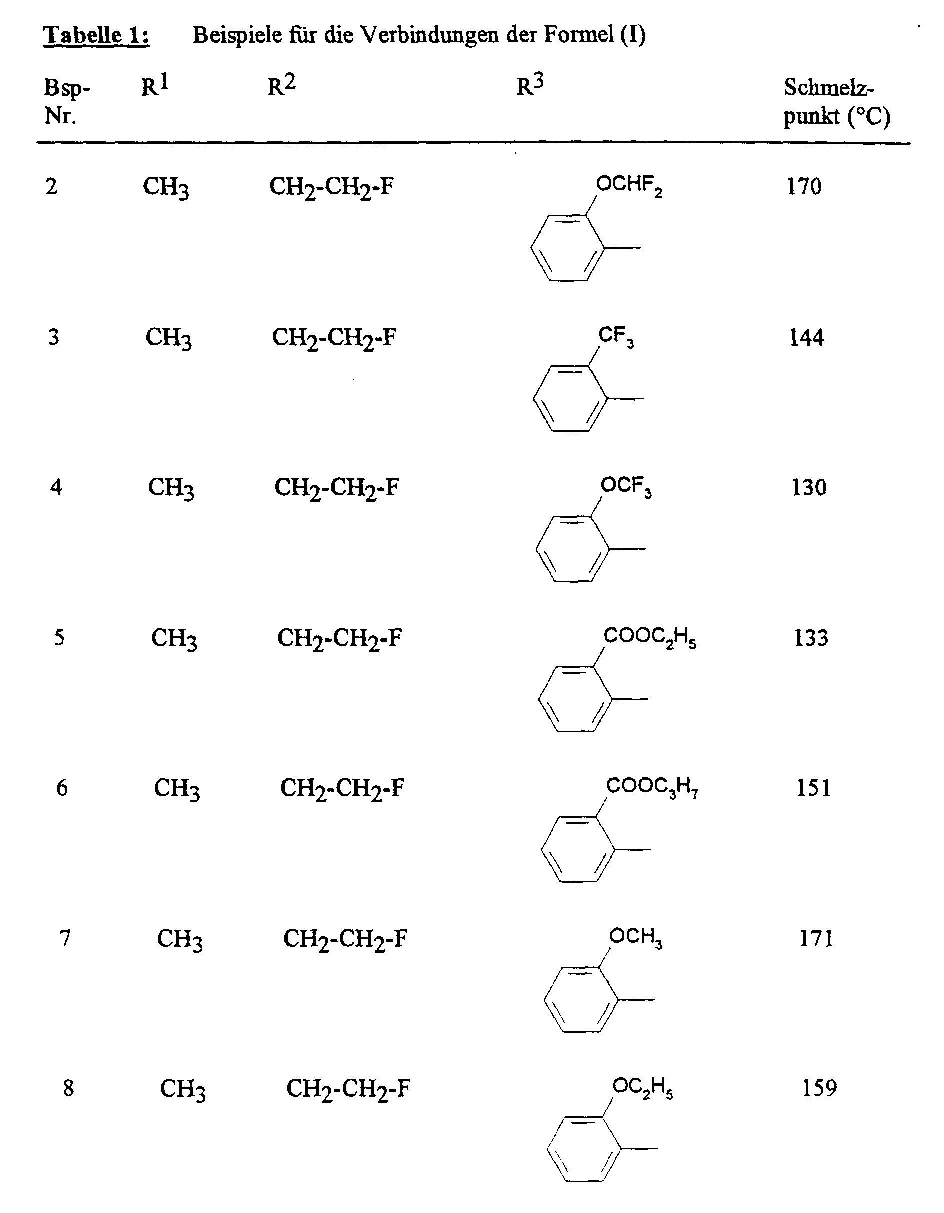

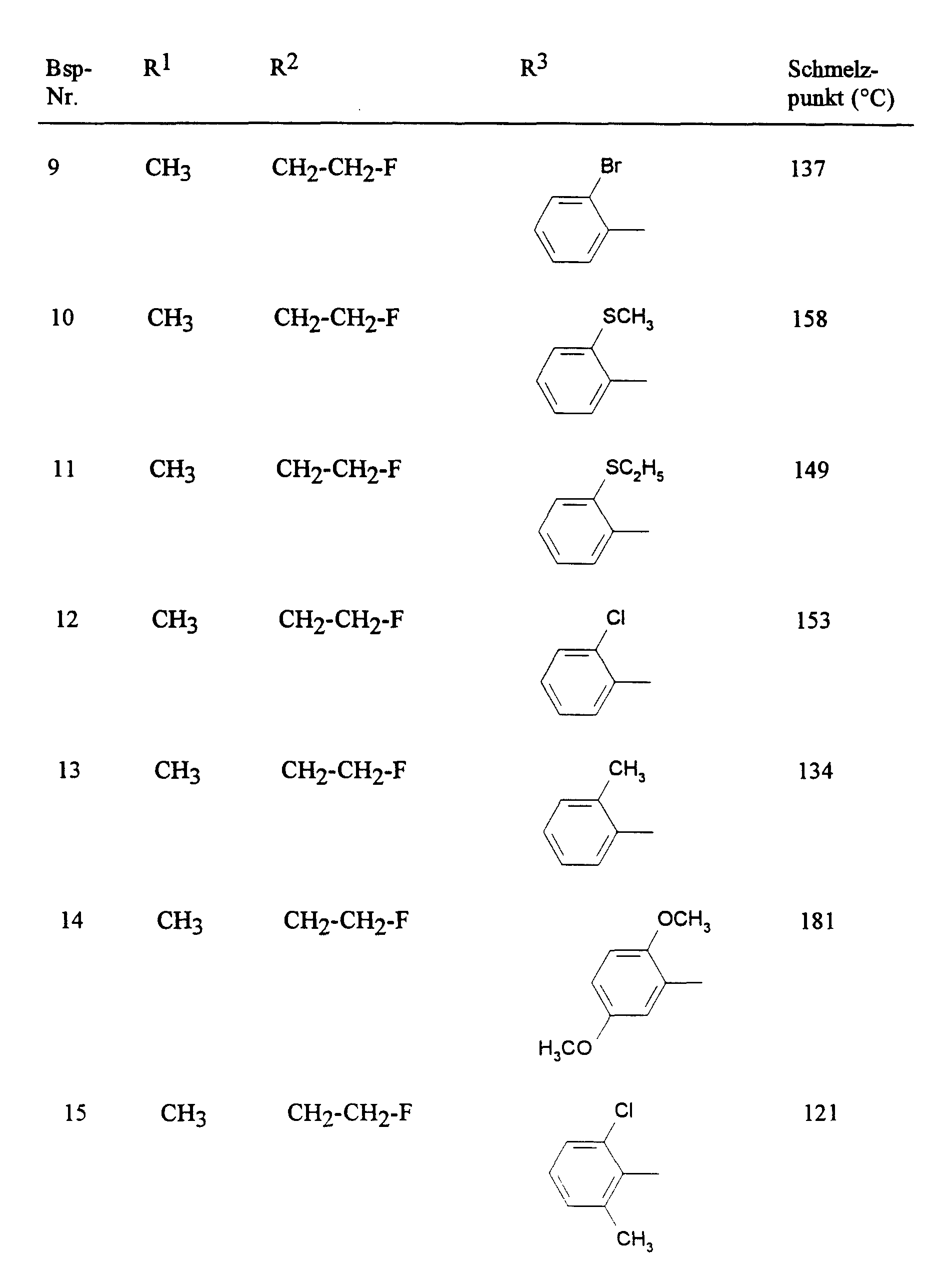

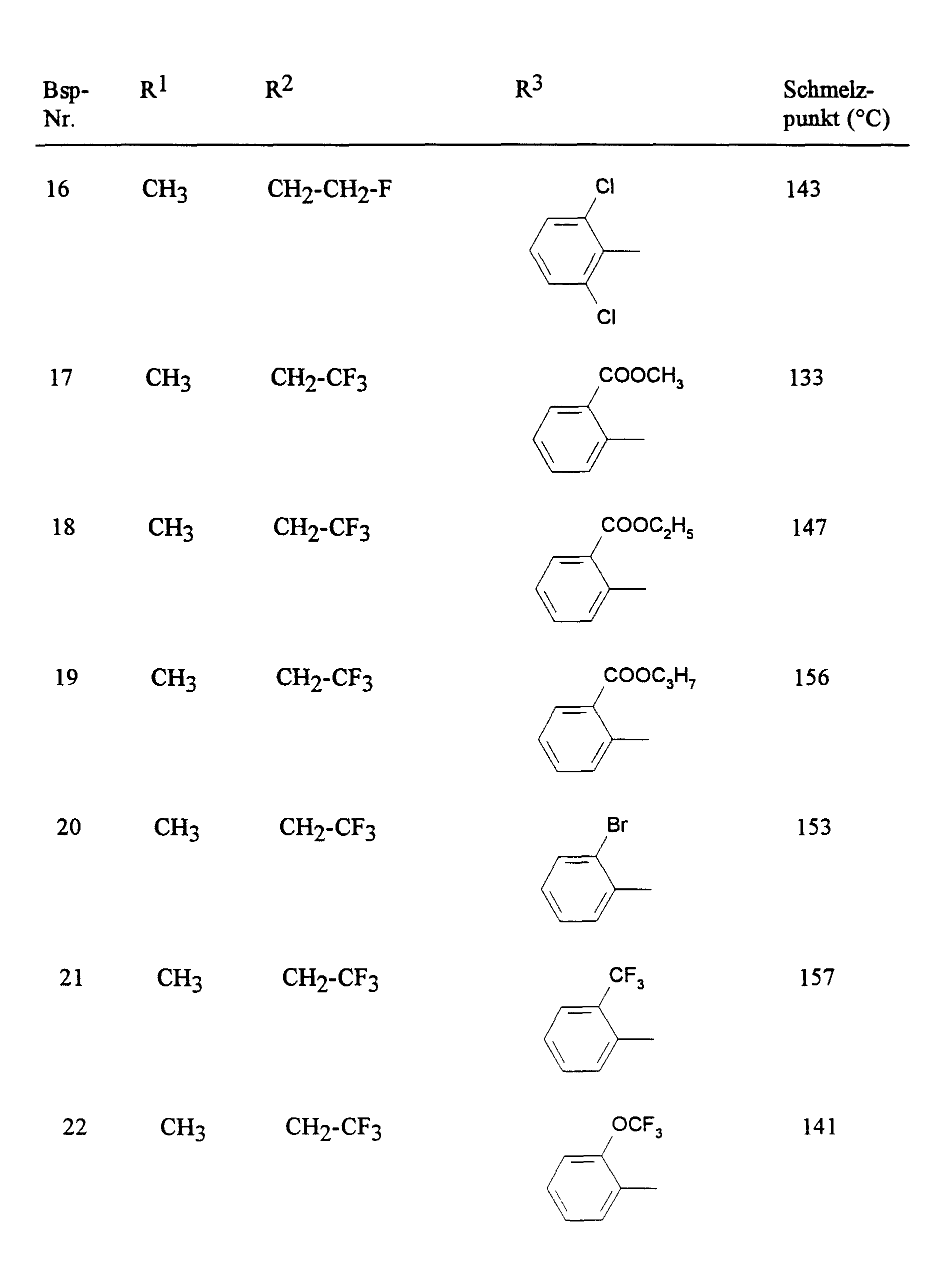

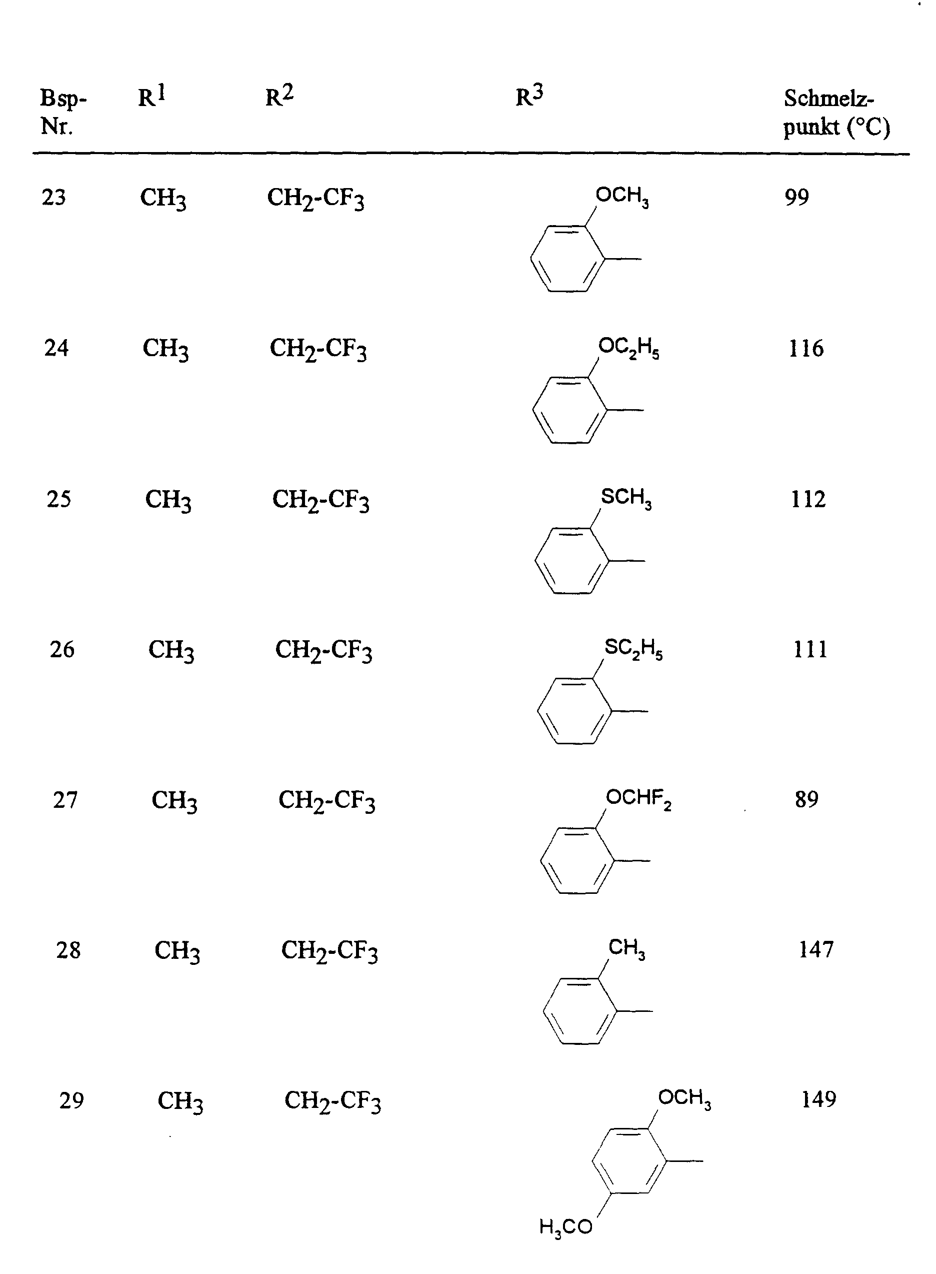

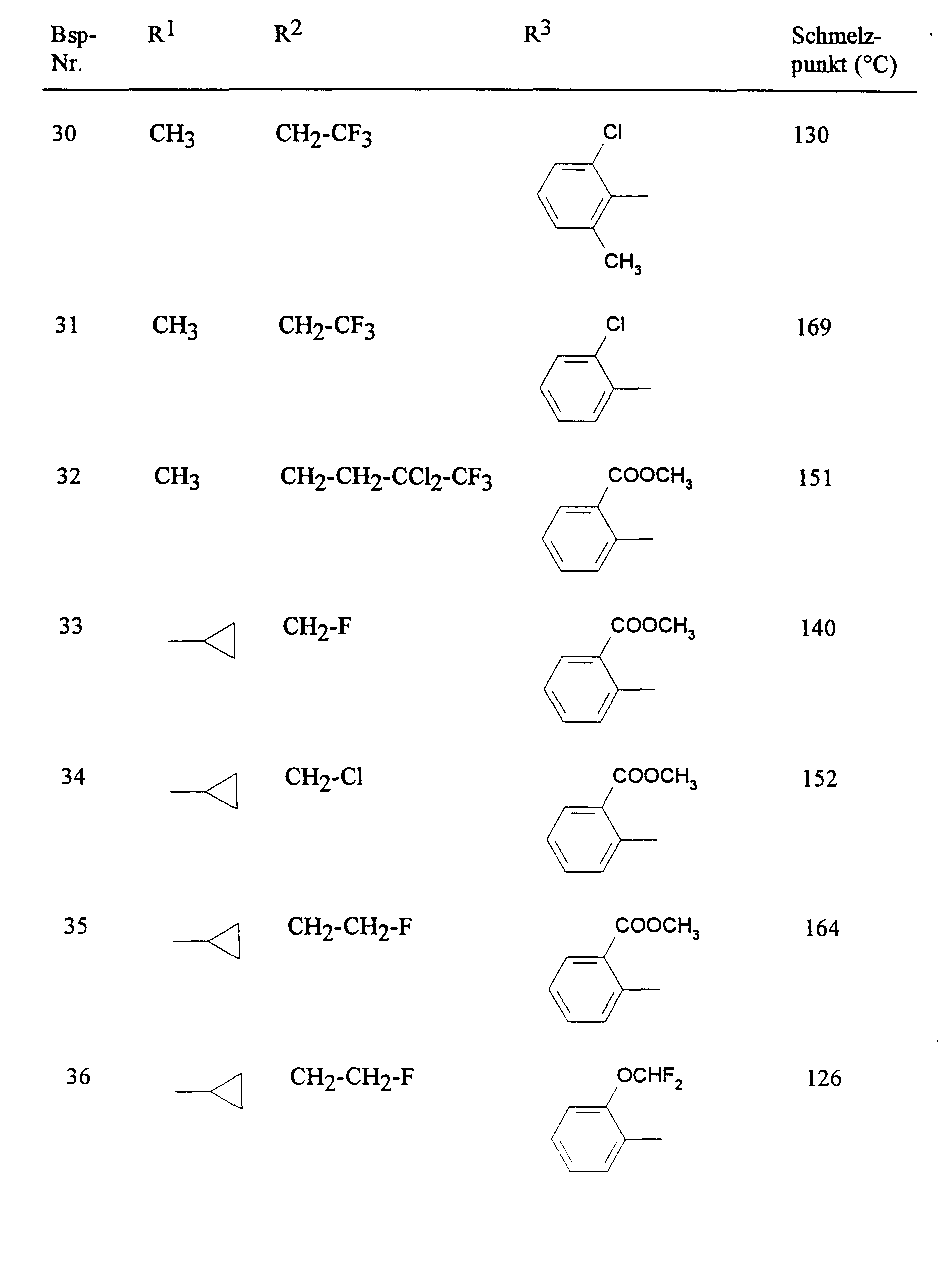

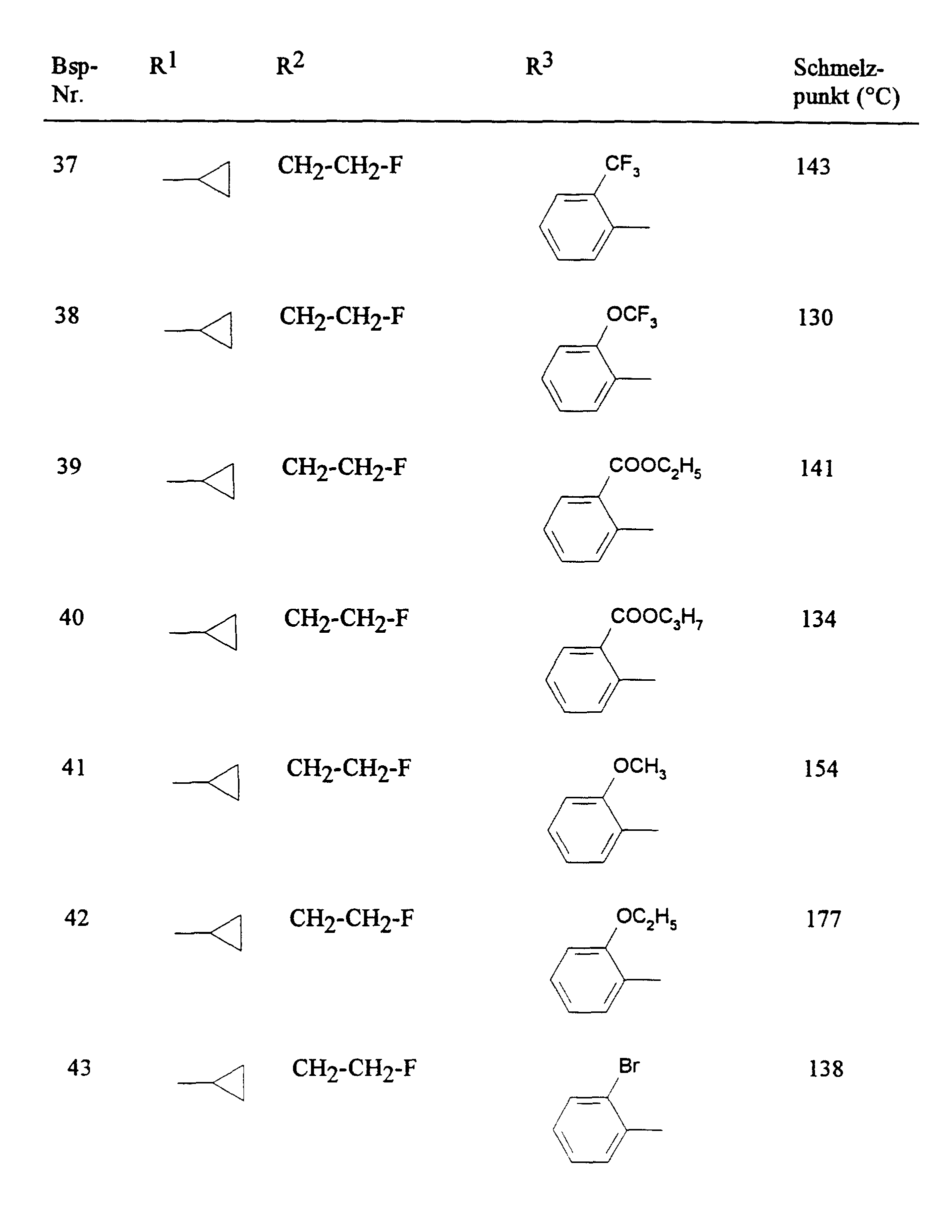

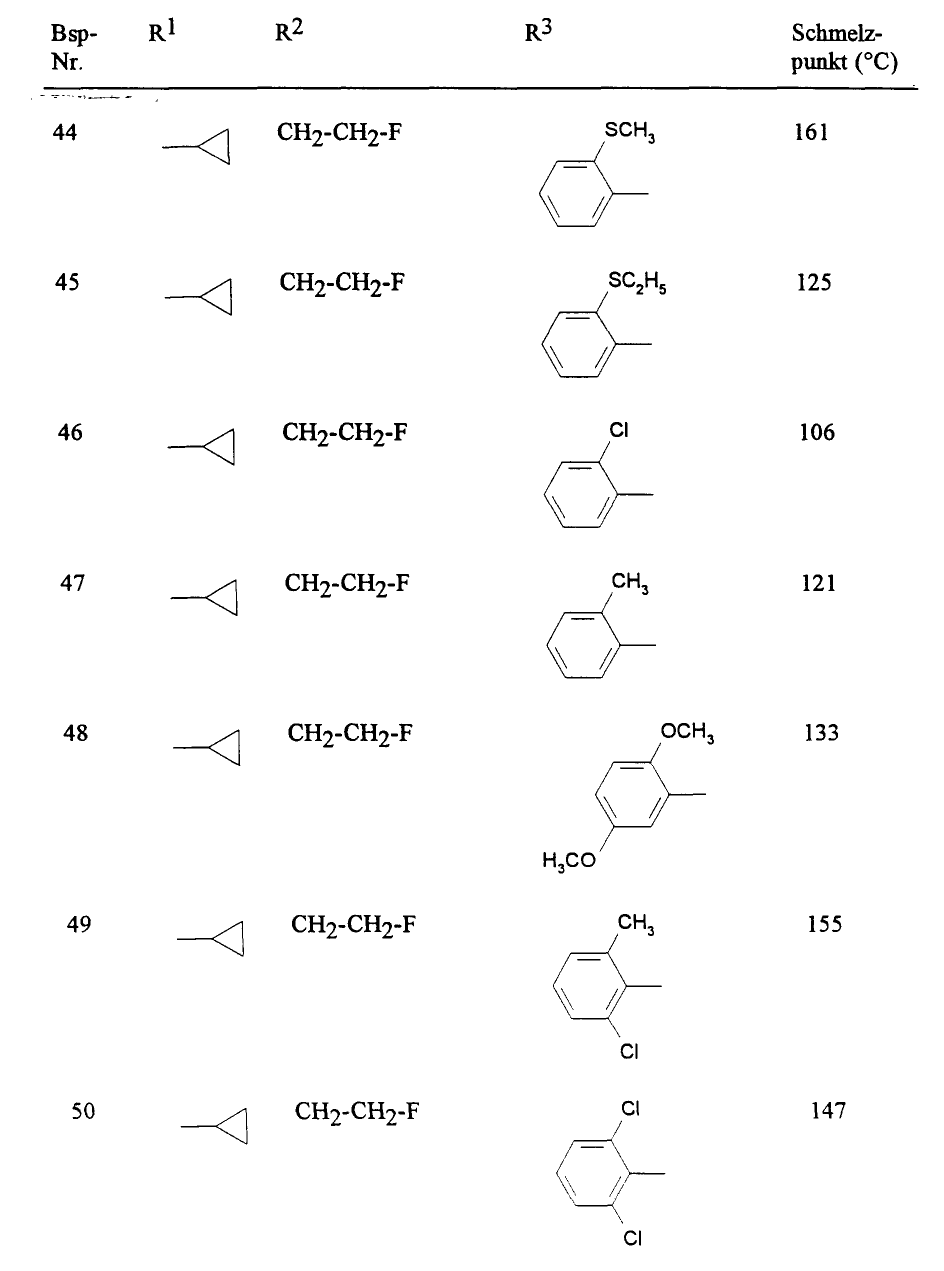

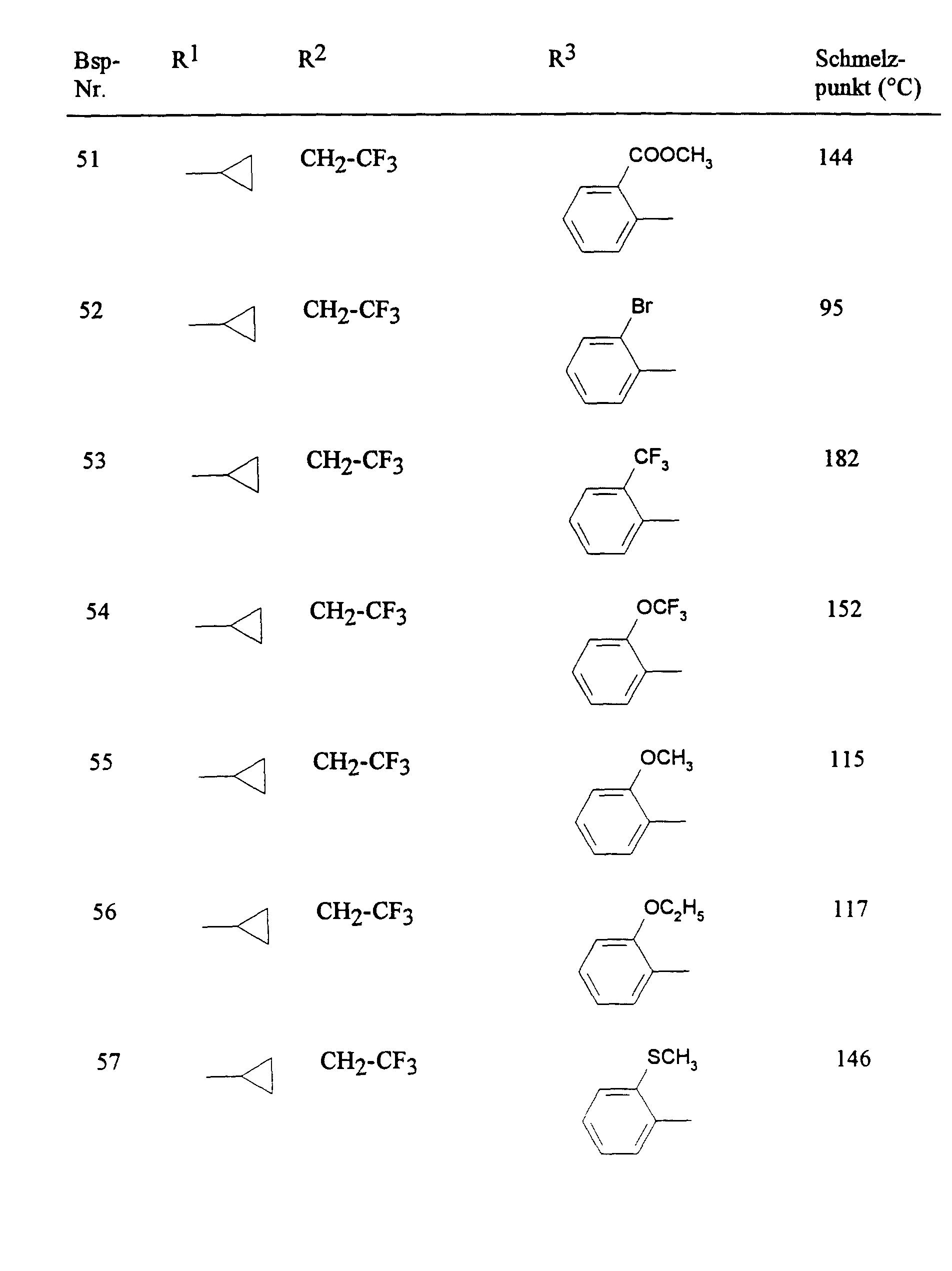

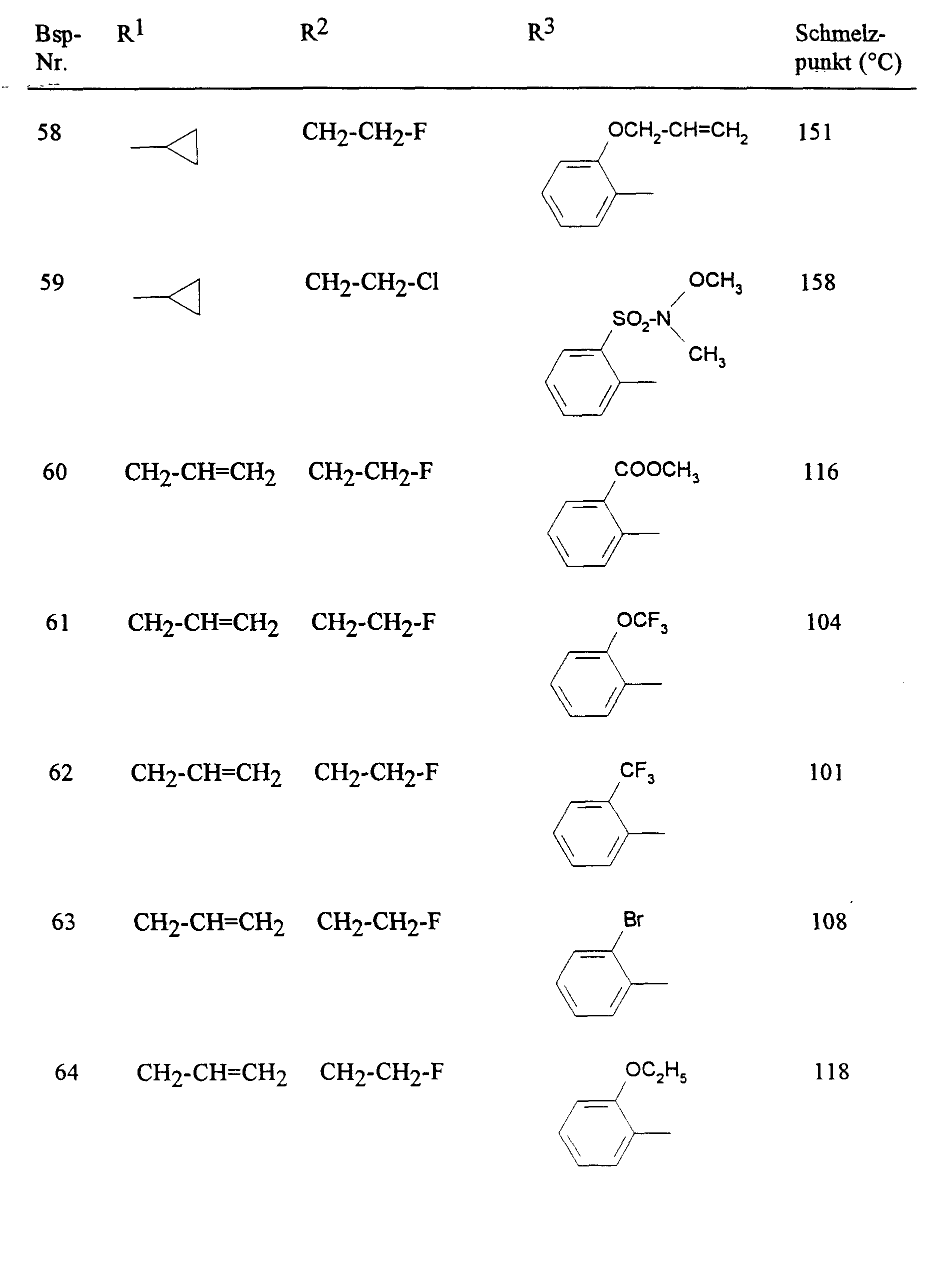

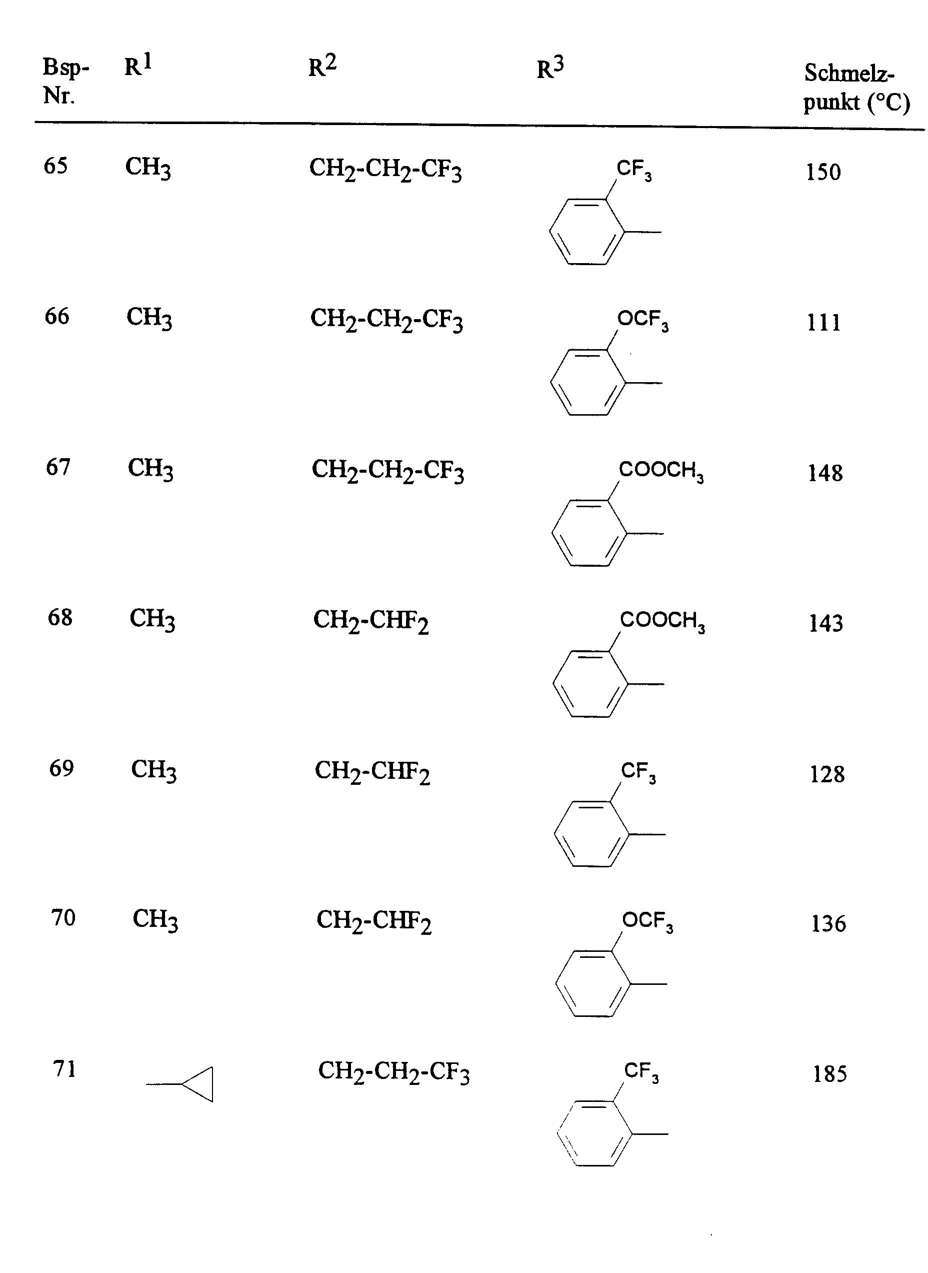

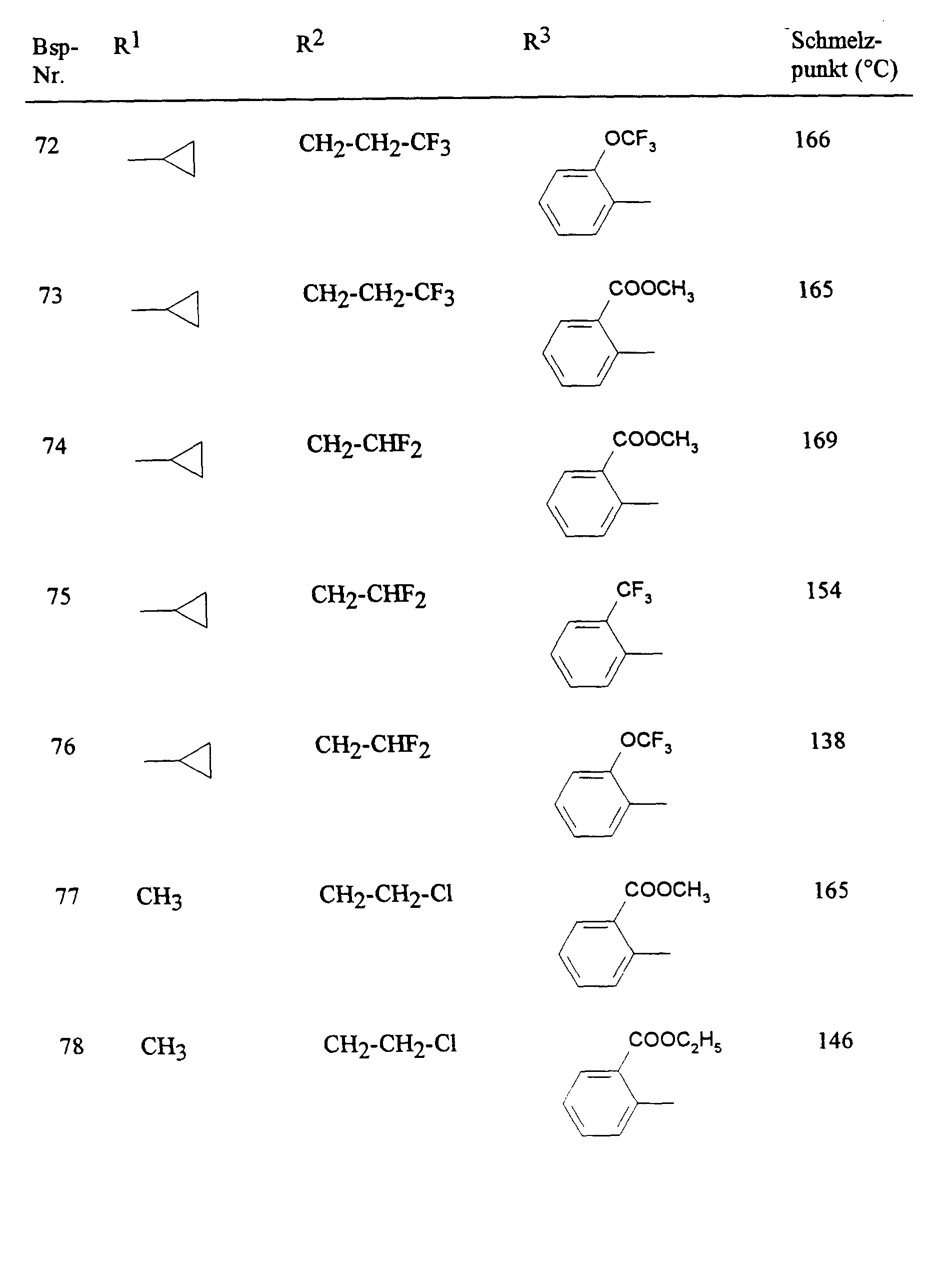

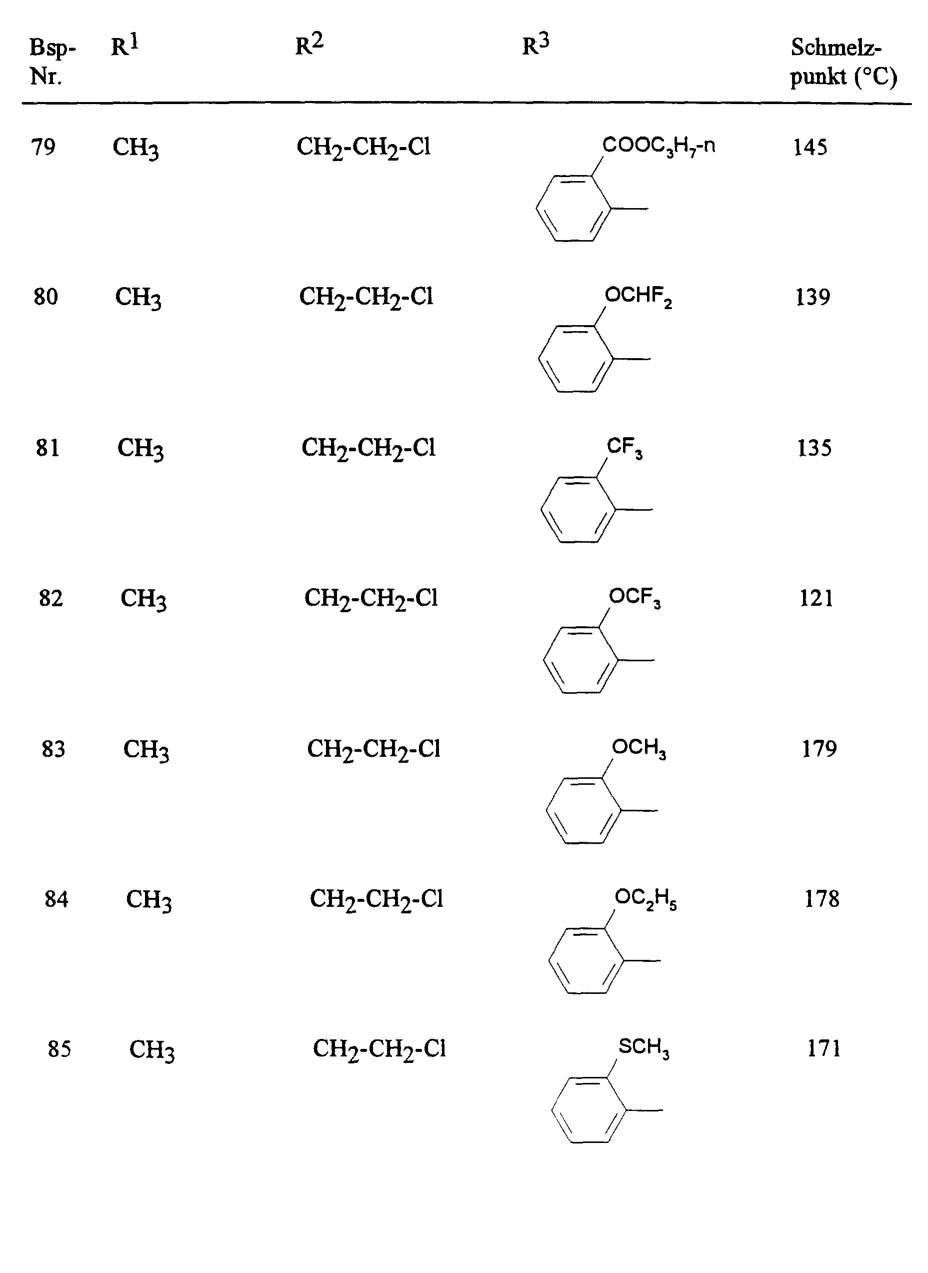

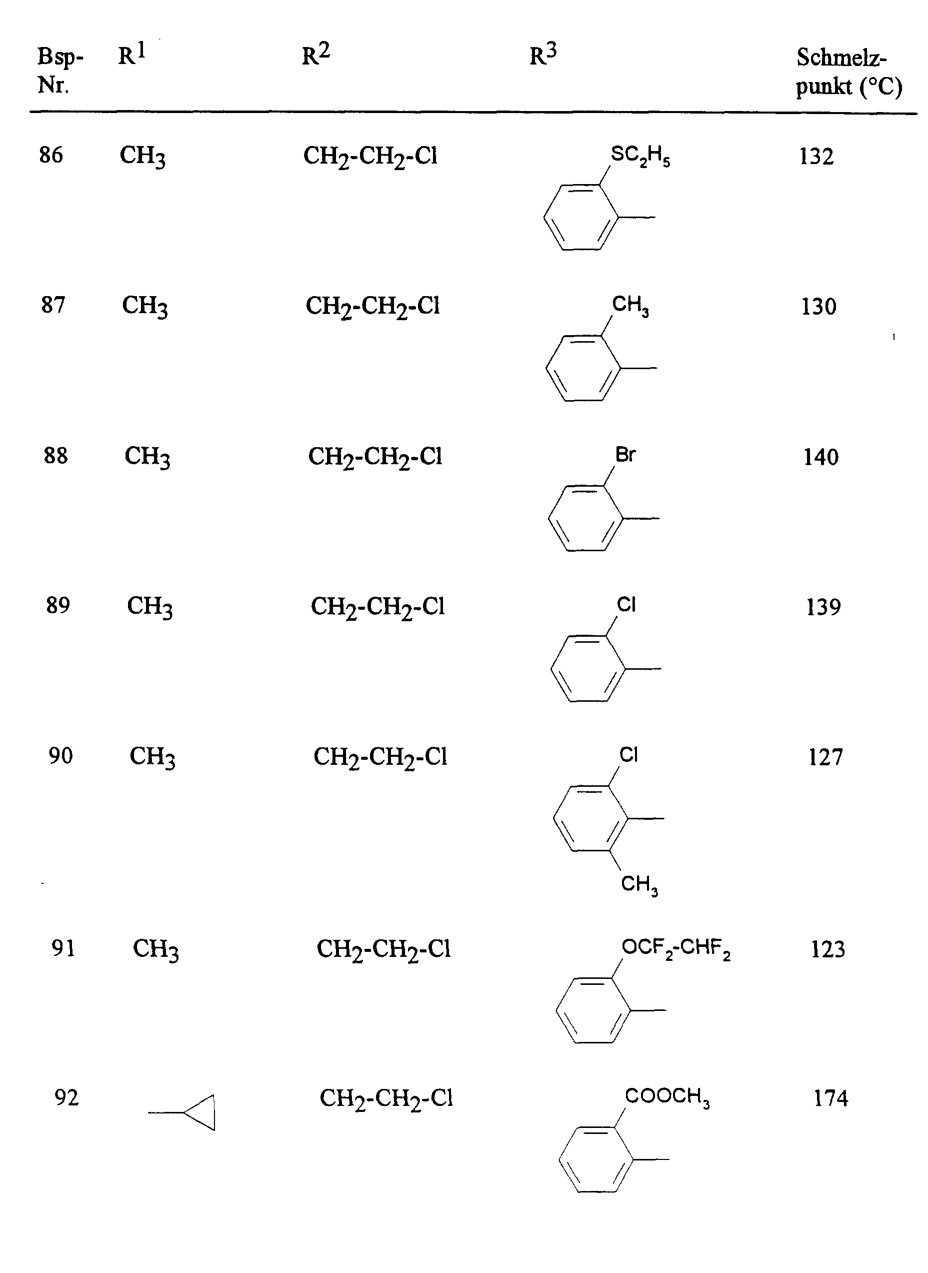

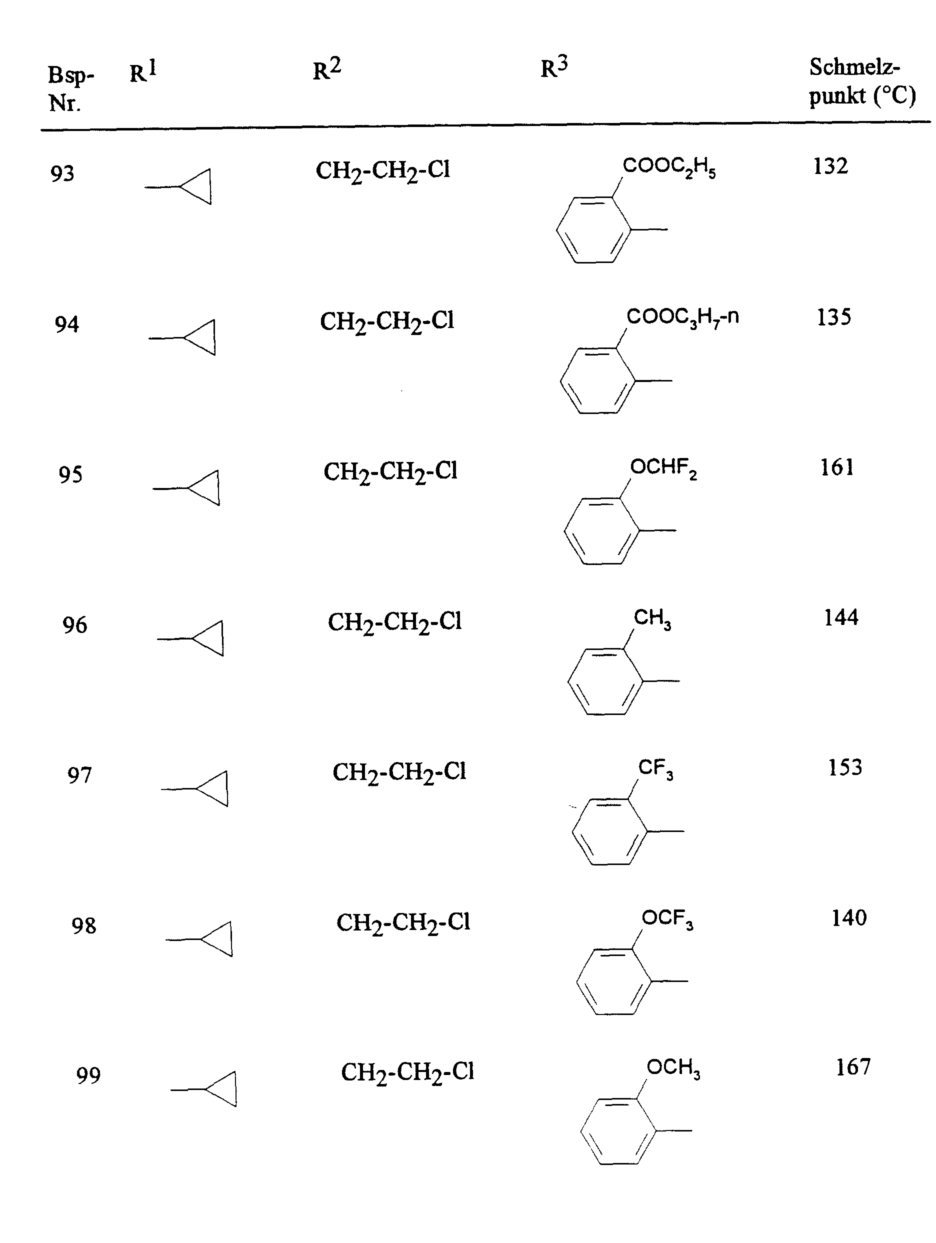

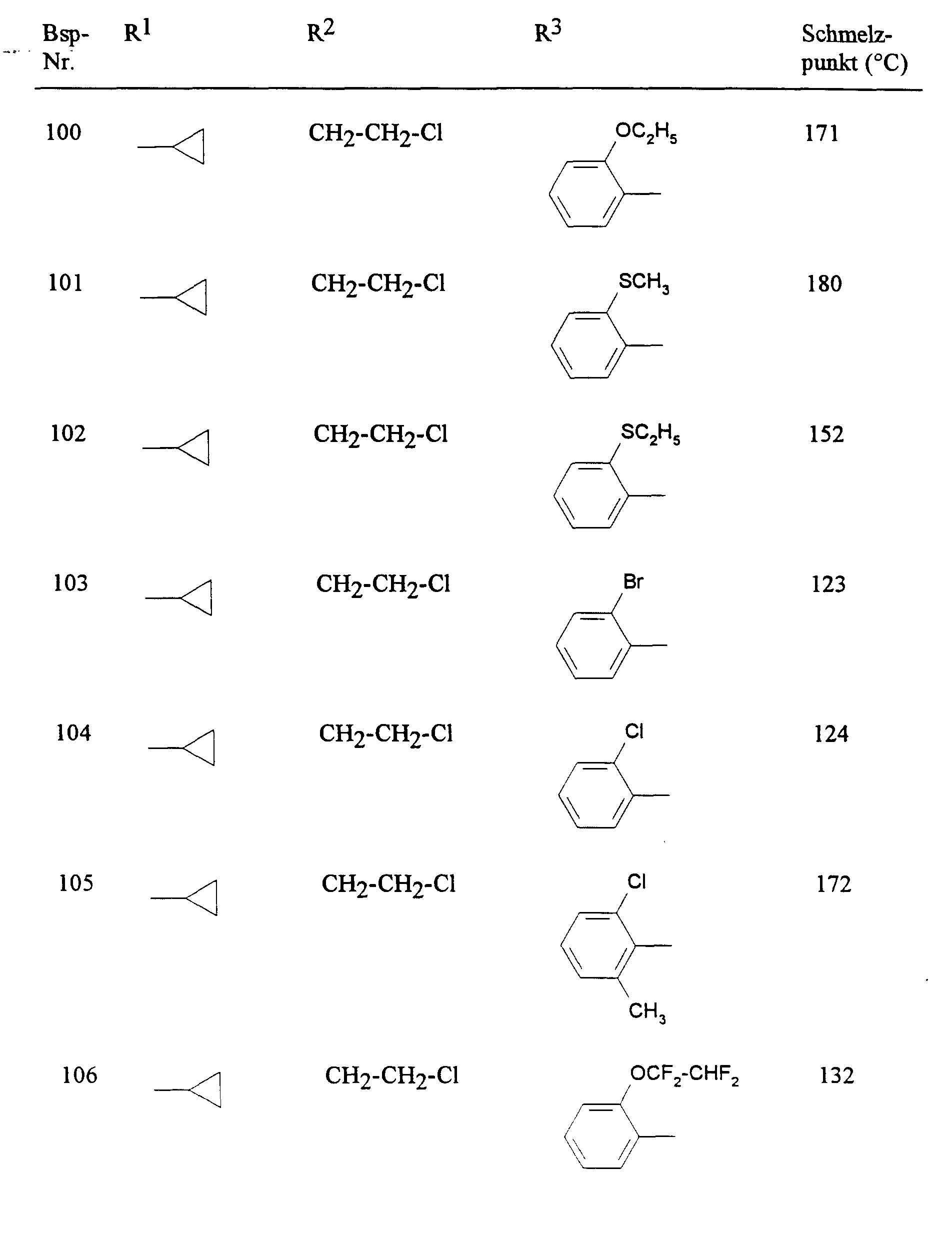

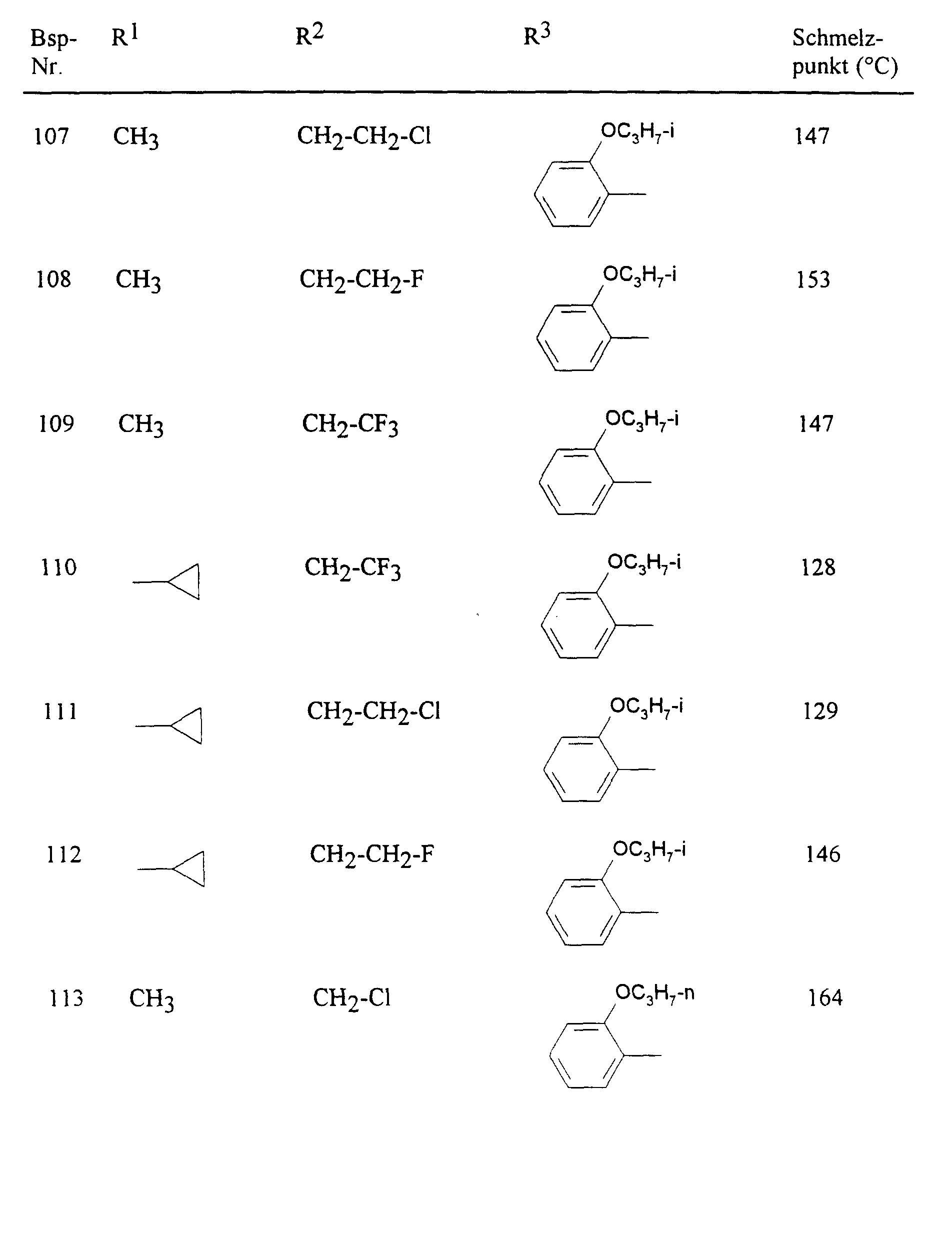

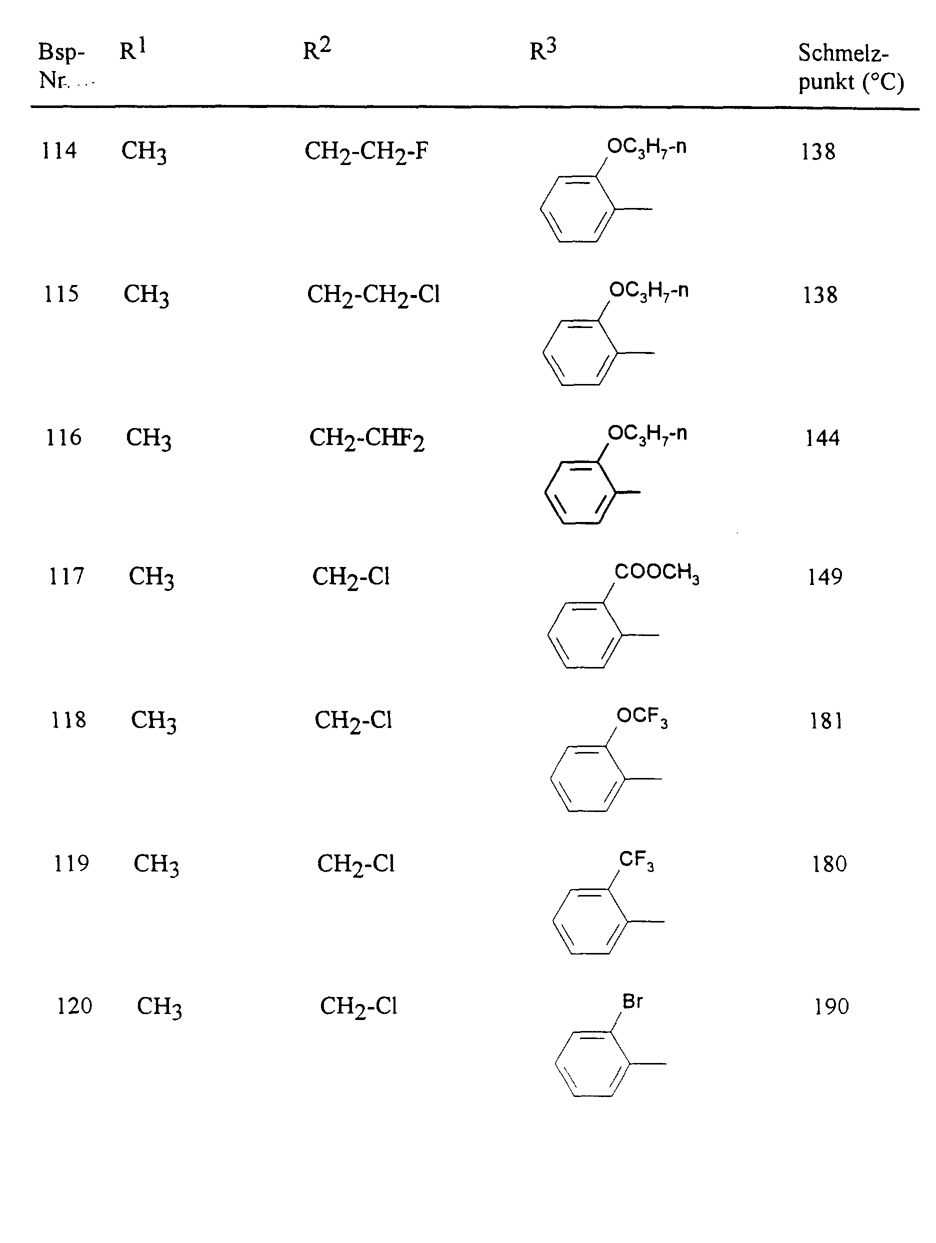

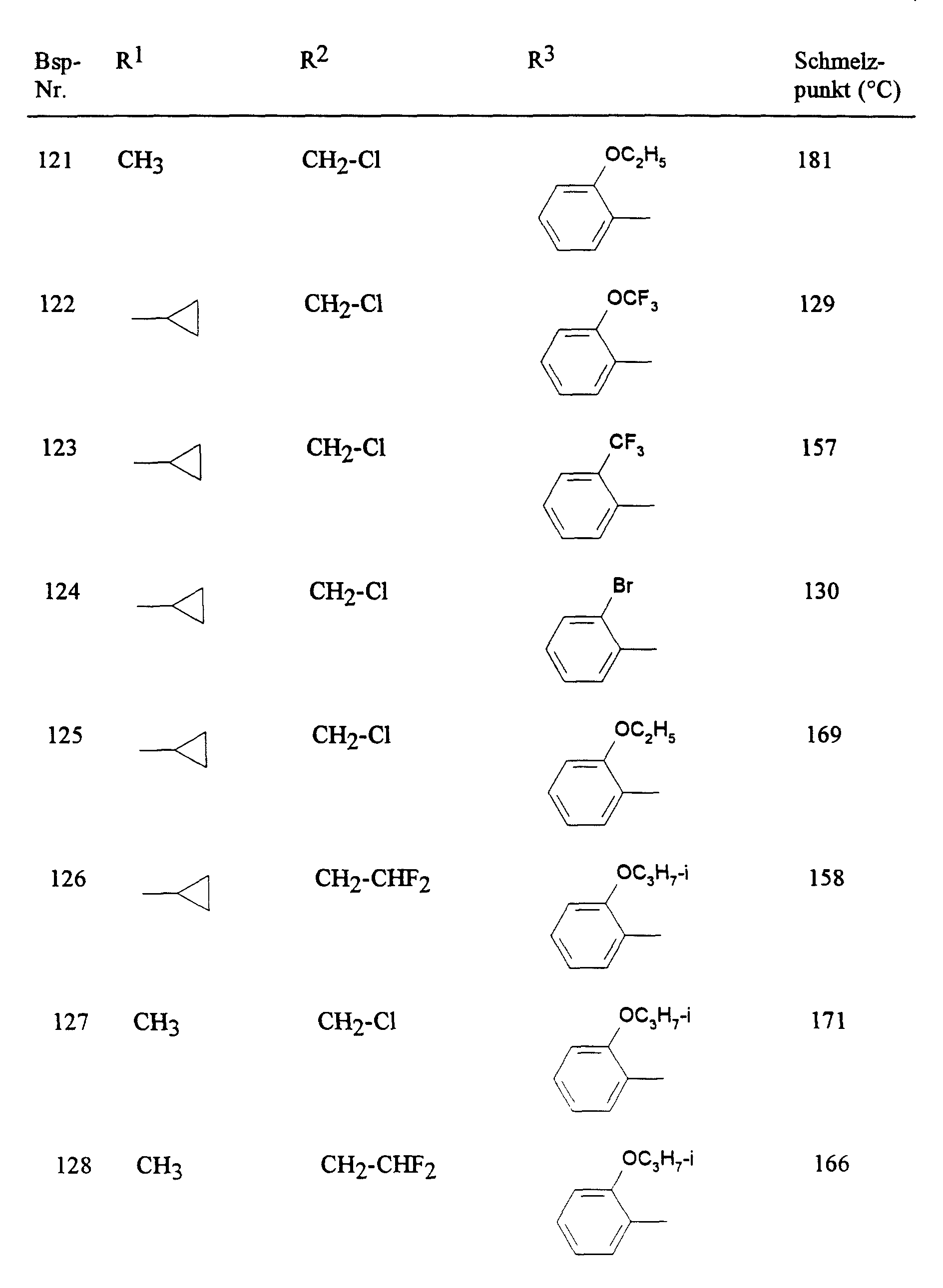

Die in Tabelle 1 als Beispiel 58 aufgeführte Verbindung kann beispielsweise wie folgt hergestellt werden:

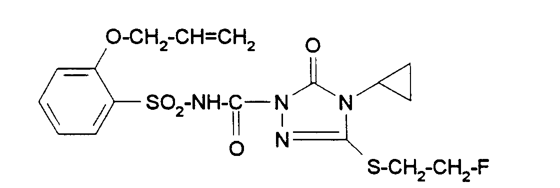

### (Verfahren (b))

1,07 g (5 mMol) 2-Allyloxy-benzolsulfonamid werden in 25 ml Acetonitril gelöst und mit 0,8 g (5,3 mMol) Diazabicycloundecen (DBU) unter Rühren versetzt. Nach 30 Minuten bei 20°C werden 1,62 g (5 mMol) 4-Cyclopropyl-5-(2-fluor-ethylthio)-2-phenoxycarbonyl-2,4-dihydro-3H-1,2,4-triazol-3-on zugegeben und die Mischung wird weiter zwölf Stunden gerührt. Dann wird der Reaktionsansatz auf Eiswasser gegossen, mit 10%iger Salzsäure angesäuert und zweimal mit Methylenchlorid extrahiert. Nach Trocknen mit Magnesiumsulfat und Einengen im Vakuum wird der verbliebene Rückstand mit Ether zur Kristallisation gebracht.

Man erhält 1,4 g (63% der Theorie) 2-(2-Allyloxy-phenylsulfonyl-aminocarbonyl)-4-cyclopropyl-5-(2-fluor-ethylthio)-2,4-dihydro-3H-1,2,4-triazol-3-on vom Schmelzpunkt 151°C.

Die in Tabelle 1 als Beispiel 59 aufgeführte Verbindung kann beispielsweise wie folgt hergestellt werden

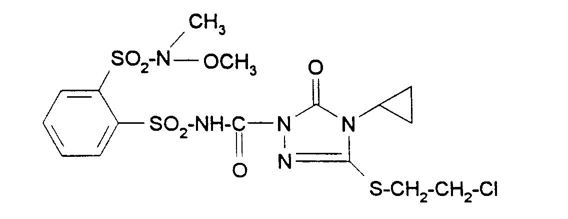

### (Verfahren (d))

Eine Mischung aus 4,4 g (20 mMol) 5-(2-Chlor-ethylthio)-4-cyclopropyl-2,4-dihydro-3H-1,2,4-triazol-3-on, 7,6 g (24 mMol) 2-(N-Methoxy-N-methyl-aminosulfonyl)-benzol-sulfochlorid, 2,6 g (40 mMol) Natrium-cyanat und 1,2 g (15 mMol) Pyridin in 50 ml Acetonitril wird 48 Stunden bei 20°C gerührt. Dann wird im Vakuum eingeengt, der ölige Rückstand in Methylenchlorid aufgenommen und mit 10%iger Salzsäure gewaschen. Die organische Phase wird mit Magnesiumsulfat getrocknet, im Vakuum eingeengt und der verbliebene Rückstand mit Methanol/Isopropanol kristallisiert.

Man erhält 4,7 g (45% der Theorie) 5-(2-Chlorethylthio)-4-cyclopropyl-2-[2-(N-methoxy-N-methyl-aminosulfonyl)-phenylsulfonyl-aminocarbonyl]-2,4-dihydro-3H-1,2,4-triazol-3-on vom Schmelzpunkt 158°C.

### Ausgangsstoffe der Formel (II)

### Beispiel (II-1)

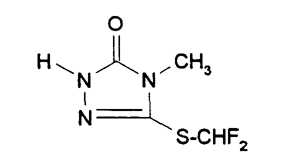

6,6 g (0,05 Mol) 5-Mercapto-4-methyl-2,4-dihydro-3H-1,2,4-triazol-3-on werden in 40 ml Wasser und 40 ml Dioxan gelöst und mit 10 g (0,25 Mol) Natriumhydroxid versetzt. Unter kräftigem Rühren wird bei 80°C für 16 Stunden ein schwacher Strom Frigen 22 (Difluorchlormethan) eingeleitet. Die erkaltete Lösung wird mit 10%iger Salzsäure schwach sauer gestellt, dann wird mehrmals mit Essigsäureethylester extrahiert. Die vereinigten organischen Phasen werden mit Magnesiumsulfat getrocknet, filtriert und im Vakuum eingeengt. Der Rückstand wird mit Diethylether zur Kristallisation gebracht.

Man erhält 0,7 g (7,7% der Theorie) 5-Difluormethylthio-4-methyl-2,4-dihydro-3H-1,2,4-triazol-3-on vom Schmelzpunkt 87°C.

### Beispiel (II-2)

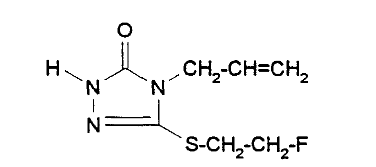

35,1 g (0,18 Mol) Kaliumsalz von 5-Mercapto-4-allyl-2,4-dihydro-3H-1,2,4-triazol-3-on werden in 200 ml Methanol gelöst, mit 22,9 g (0,18 Mol) 1-Brom-2-fluor-ethan versetzt und 12 Stunden bei 20°C gerührt. Es wird 4 Stunden bei 60°C nachgerührt, dann abgekühlt und im Vakuum eingeengt. Der Rückstand wird in Methylenchlorid aufgenommen und mit 10%iger Salzsäure und mit Wasser gewaschen. Nach Einengen im Vakuum wird der Rückstand mit Petrolether verrührt und zur Kristallisation gebracht.

Nach Absaugen und Trocknen werden 24,8 g (67,9% der Theorie) 4-Allyl-5-(2-fluorethylthio)-2,4-dihydro-3H-1,2,4-triazol-3-on vom Schmelzpunkt 65°C erhalten.

Analog m den Beispielen (II-1) und (II-2) können beispielsweise auch die in der nachstehenden Tabelle 2 aufgeführten Verbindungen der Formel (II) hergestellt werden.

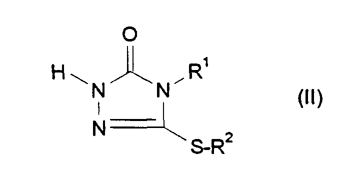

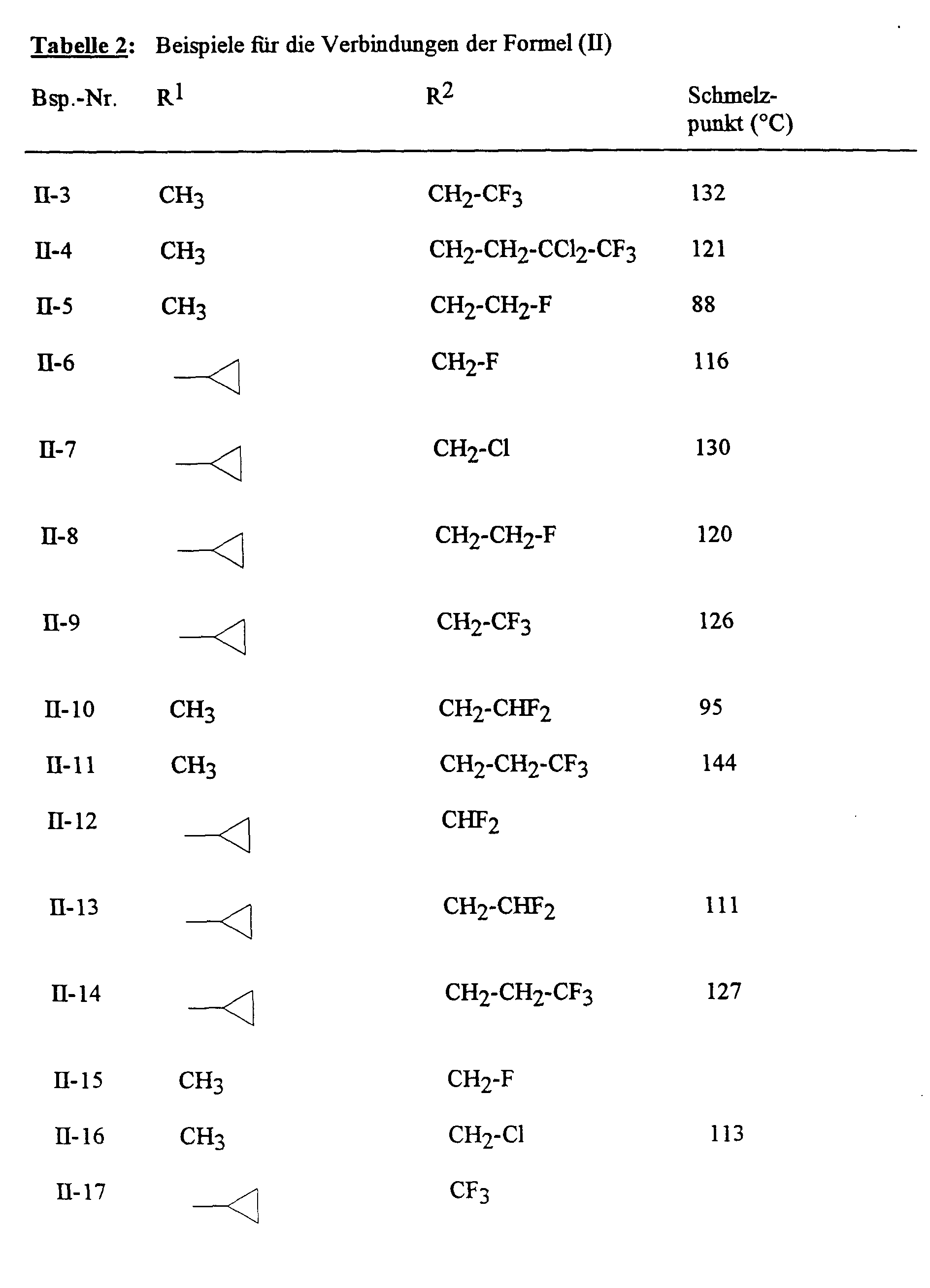

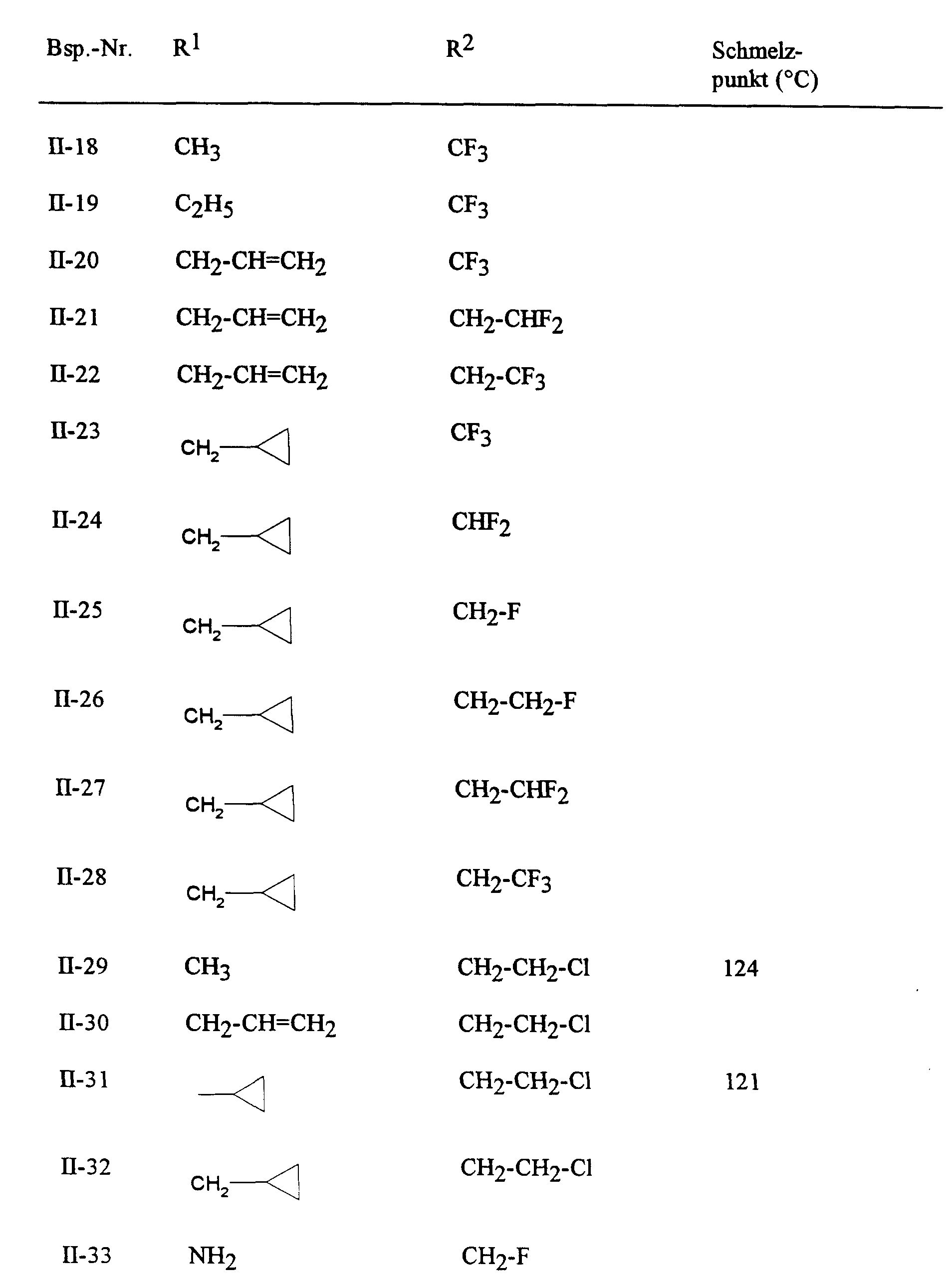

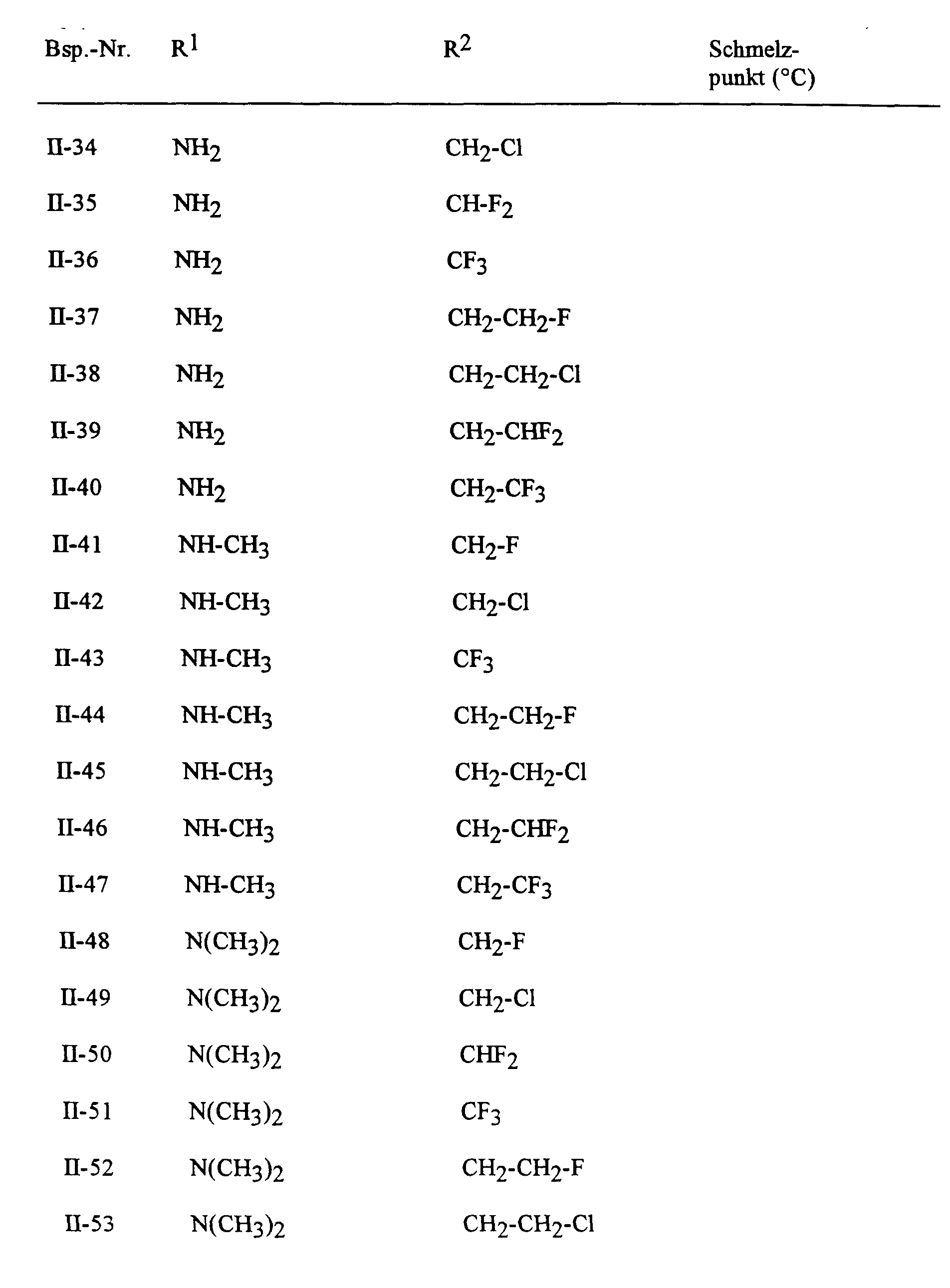

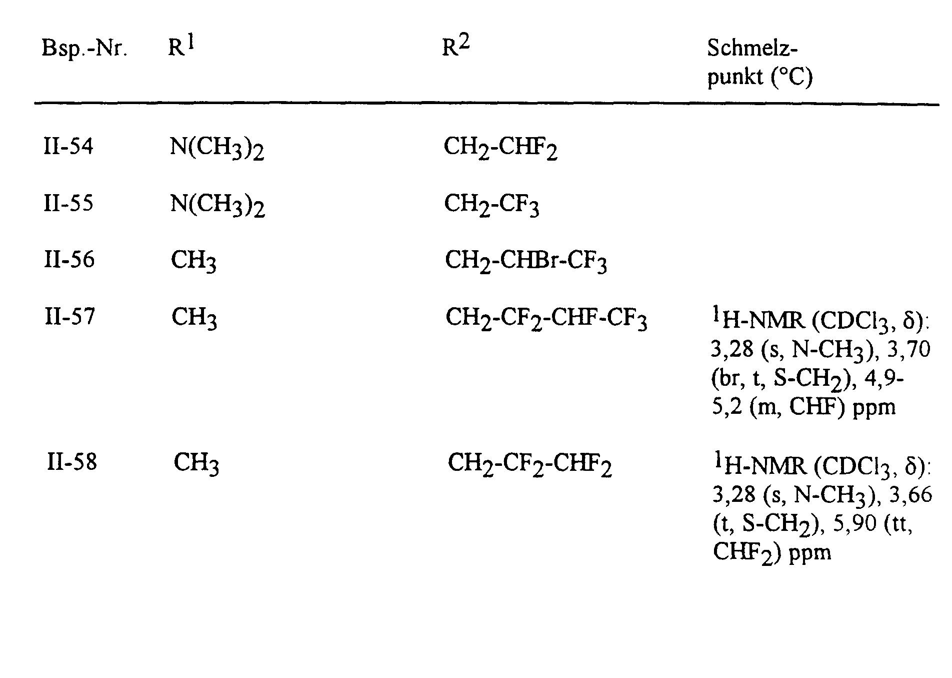

### Ausgangsstoffe der Formel (IV):

### Beispiel (IV-1)

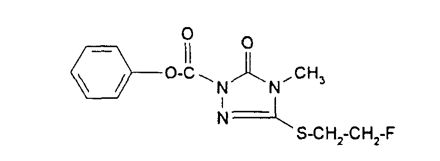

Zu einer Mischung aus 3,54 g (20 mMol) 5-(2-Fluor-ethylthio)-4-methyl-2,4-dihydro-3H-1,2,4-triazol-3-on, 0,9 g (22 mMol) Natriumhydroxid, 0,1 g (0,3 mMol) Tetrabutylammonium-bromid, 30 ml Wasser und 40 ml Methylenchlorid werden unter gutem Rühren 3,44 g (22 mMol) Chlorameisensäure-phenylester zugetropft und noch zwölf Stunden weitergerührt. Die organische Phase wird abgetrennt, mit Wasser gewaschen, mit Magnesiumsulfat getrocknet, im Vakuum eingeengt und der verbliebene Rückstand mit Ether zur Kristallisation gebracht.

Man erhält 5,1 g (86% der Theorie) 5-(2-Fluor-ethylthio)-4-methyl-2-phenoxy-carbonyl-2,4-dihydro-3H-1,2,4-triazol-3-on vom Schmelzpunkt 106°C.

Analog Beispiel (IV-1) können beispielsweise auch die in der nachstehenden Tabelle 3 aufgeführten Verbindungen der Formel (IV) hergestellt werden:

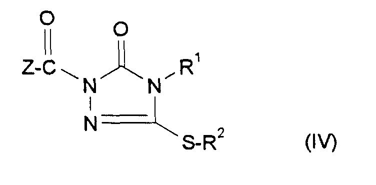

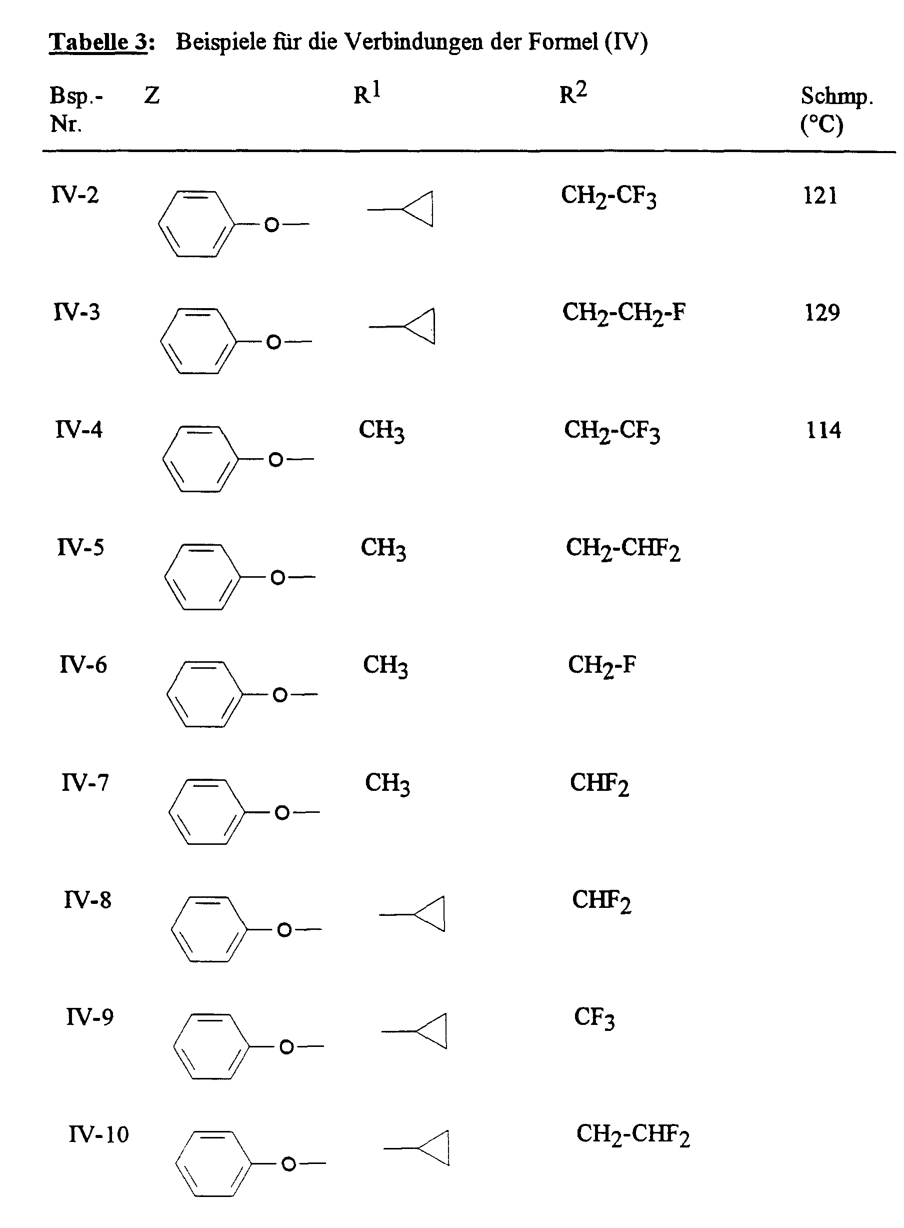

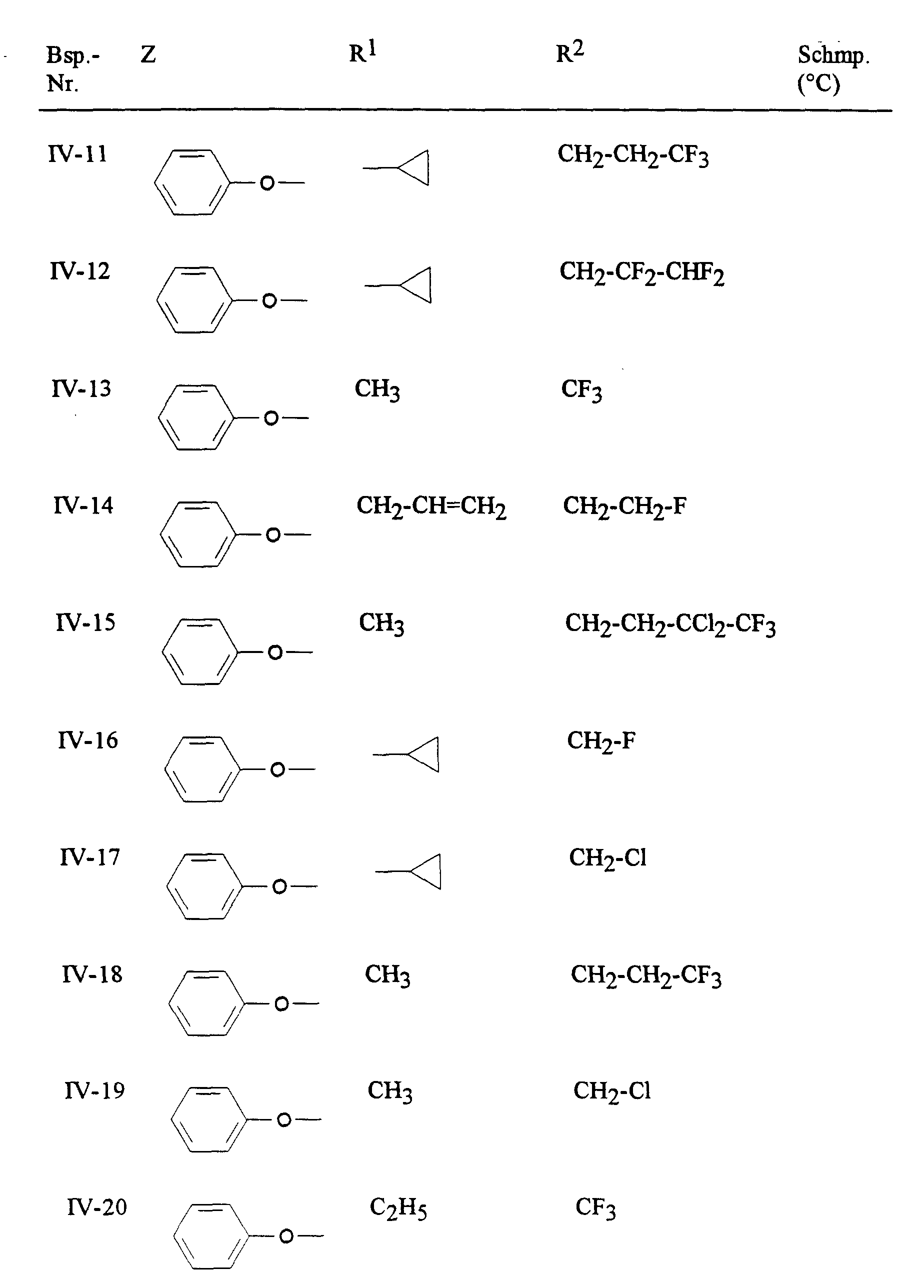

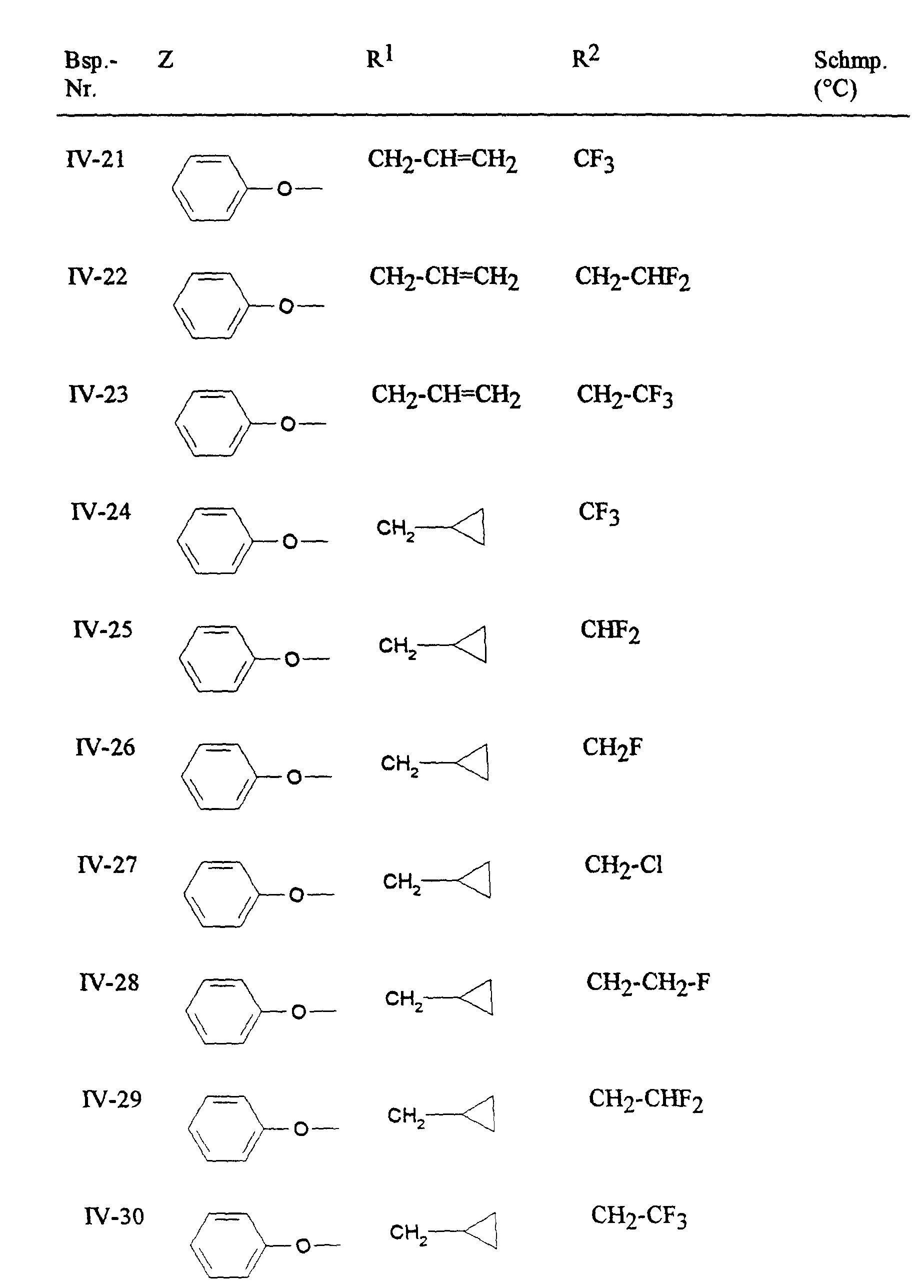

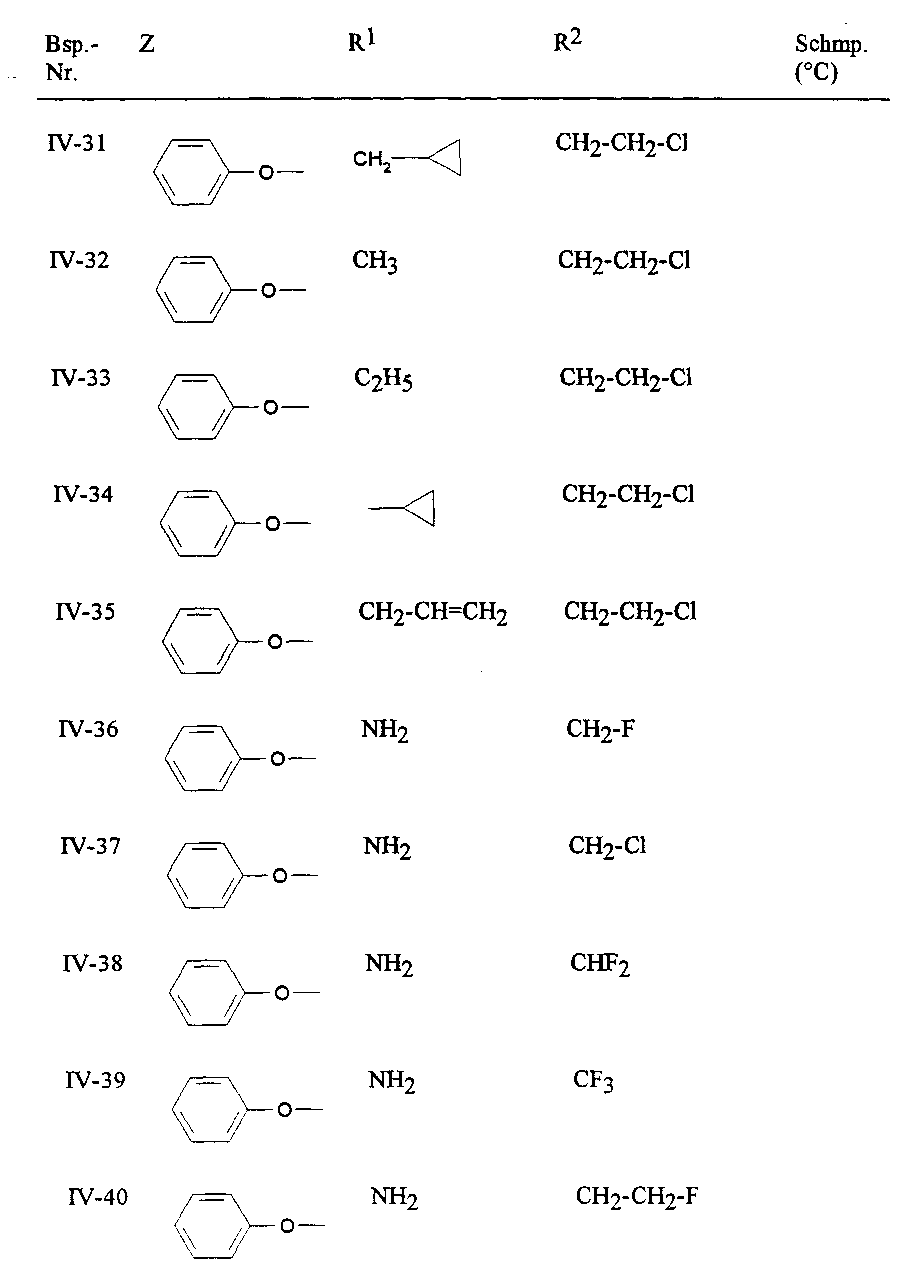

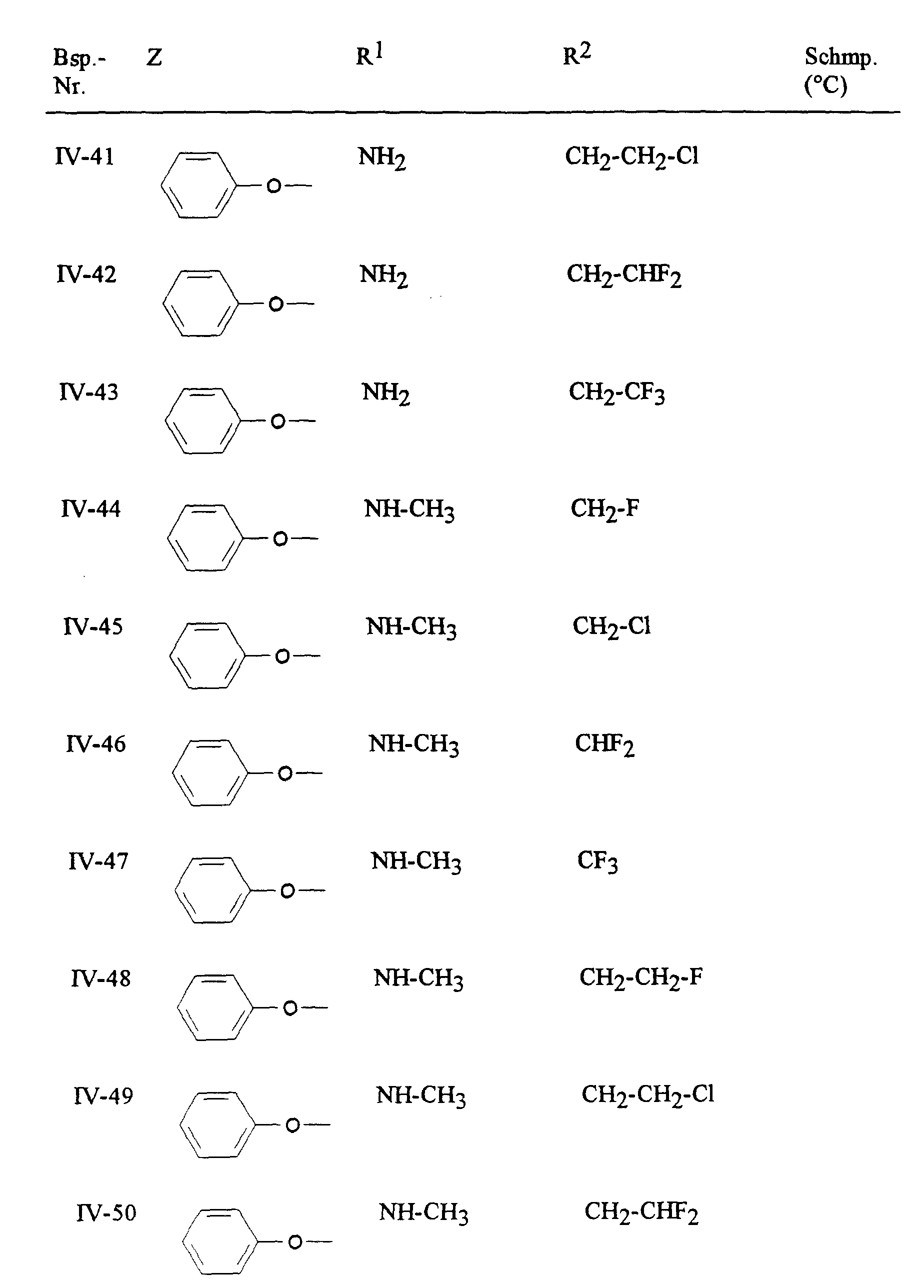

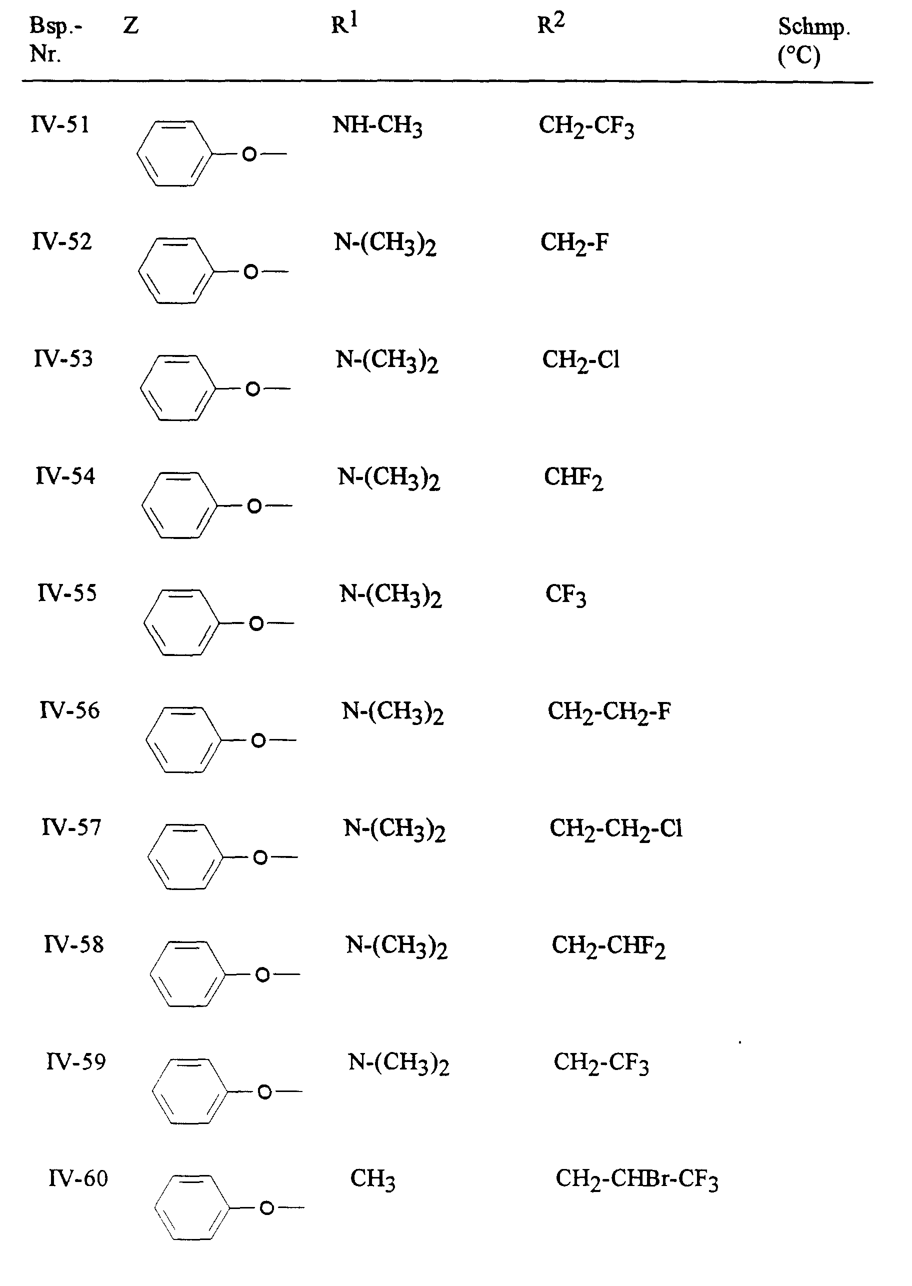

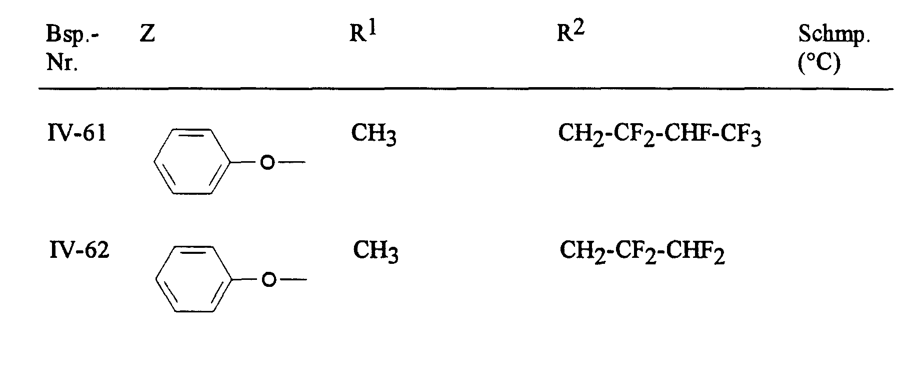

### Anwendungsbeispiele:

### Beispiel A

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 5, 11, 17, 18, 21, 25, 33, 34, 35, 36, 37, 38, 39, 40, 43, 48, 49, 51, 52, 53 und 54 starke Wirkung gegen Unkräuter.

**Tabelle A:**

| Pre-emergence-Test/Gewächshaus | | | | | | | | |
|---|---|---|---|---|---|---|---|---|
| Verbindung gemäß Herstellungs-beispiel Nr. | Aufwandmenge g/ha | Bromus | Poa | Amaranthus | Galinsoga | Matricaria | Portulaca | Viola |
| 51 | 250 | 95 | 95 | 95 | 95 | 100 | 95 | 95 |
| 34 | 125 | 80 | 95 | 95 | 95 | 100 | 100 | 90 |
| 52 | 125 | 80 | - | 90 | 95 | 90 | 60 | - |
| 53 | 125 | 90 | - | 90 | 95 | 90 | 80 | 80 |
| 54 | 125 | 90 | - | 90 | 95 | 80 | - | 60 |
| 33 | 125 | 95 | 95 | 95 | 95 | 95 | 95 | 90 |
| 17 | 125 | 95 | 95 | 95 | 95 | 95 | 100 | 95 |
| 18 | 125 | 95 | 90 | 80 | 90 | 60 | - | 95 |
| 21 | 125 | 80 | 80 | 90 | 95 | 95 | 90 | 95 |
| 25 | 125 | 95 | 90 | 95 | 95 | 95 | 100 | 90 |
| 5 | 125 | 95 | 95 | 95 | 95 | 80 | 95 | 90 |
| 11 | 125 | 80 | 70 | 95 | 90 | 95 | 95 | 95 |
| 35 | 125 | 95 | 95 | 95 | 95 | 95 | 95 | 95 |
| 36 | 125 | 50 | 80 | 95 | 95 | - | 95 | 90 |
| 37 | 125 | 95 | 60 | 95 | 100 | 95 | 100 | 95 |
| 38 | 125 | 80 | 60 | 95 | 100 | 100 | 90 | 95 |
| 39 | 125 | 95 | 95 | 95 | 100 | 95 | 100 | 95 |
| 40 | 125 | 60 | - | 95 | 95 | 80 | 80 | - |
| 43 | 125 | 80 | 70 | 95 | 100 | 95 | 95 | 95 |
| 48 | 125 | 95 | 95 | 95 | 95 | 95 | 95 | 70 |
| 49 | 125 | 90 | 95 | 95 | 95 | 95 | 80 | 90 |

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 5, 8, 10, 11, 17, 33, 34, 38, 39, 48, 49, 51, 52, 53 und 54 starke Wirkung gegen Unkräuter.

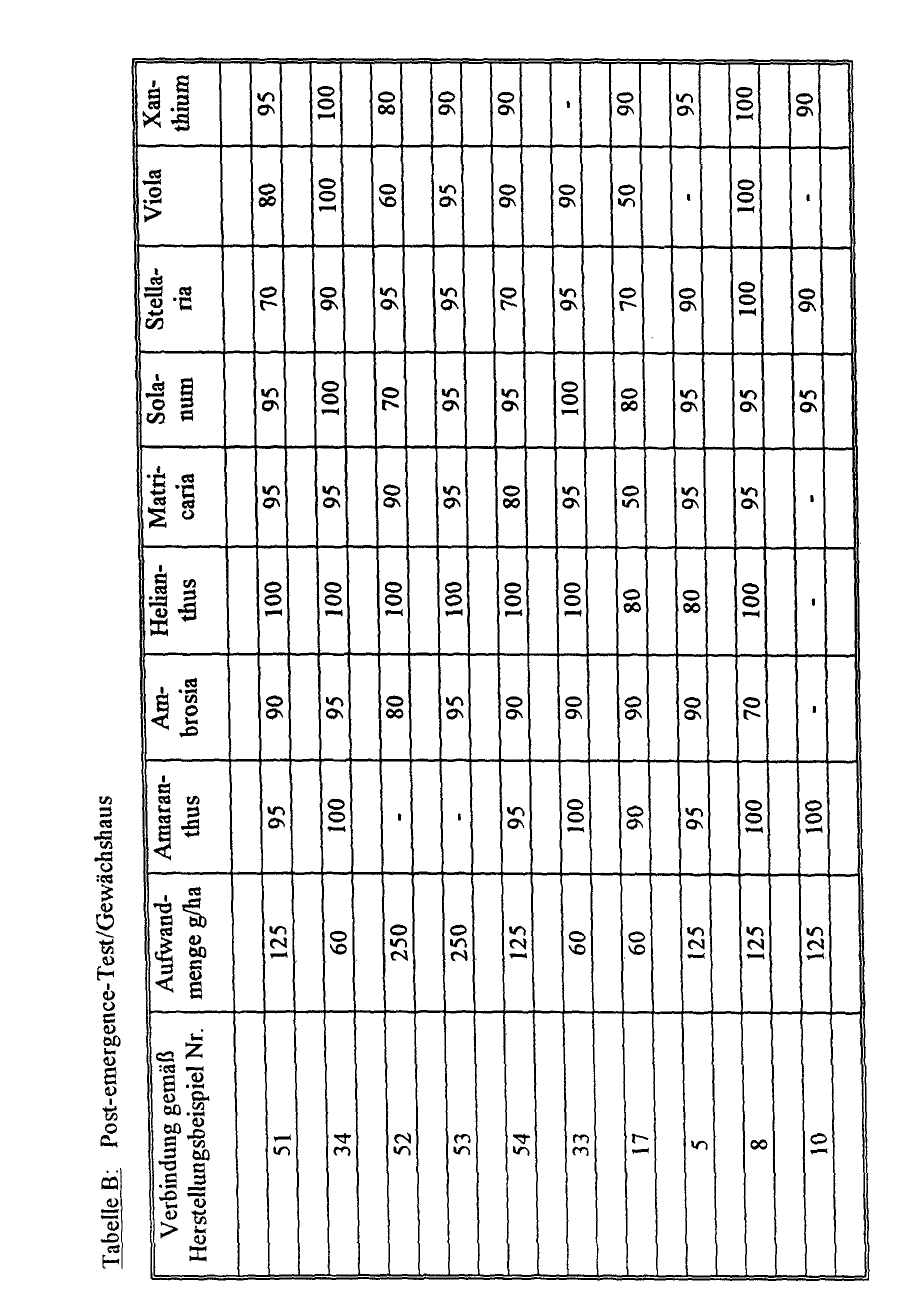

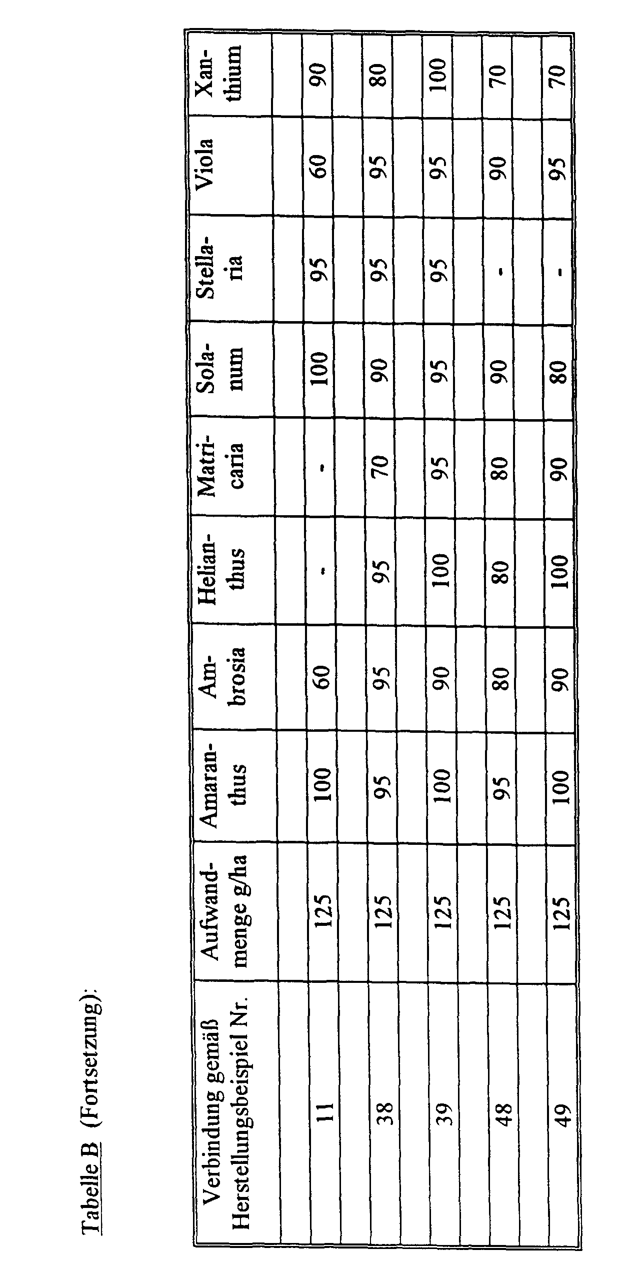

### Beispiel C

**Podosphaera-Test (Apfel)** / protektiv
- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca 70 % aufgestellt.

10 Tage nach der Inokulation erfolgt die Auswertung.

In diesem Test zeigen z.B. die Verbindungen gemäß Herstellungsbeispielen 34 und 51 bei einer Wirkstoffkonzentration von 100 ppm einen Wirkungsgrad von 98 bis 100%.

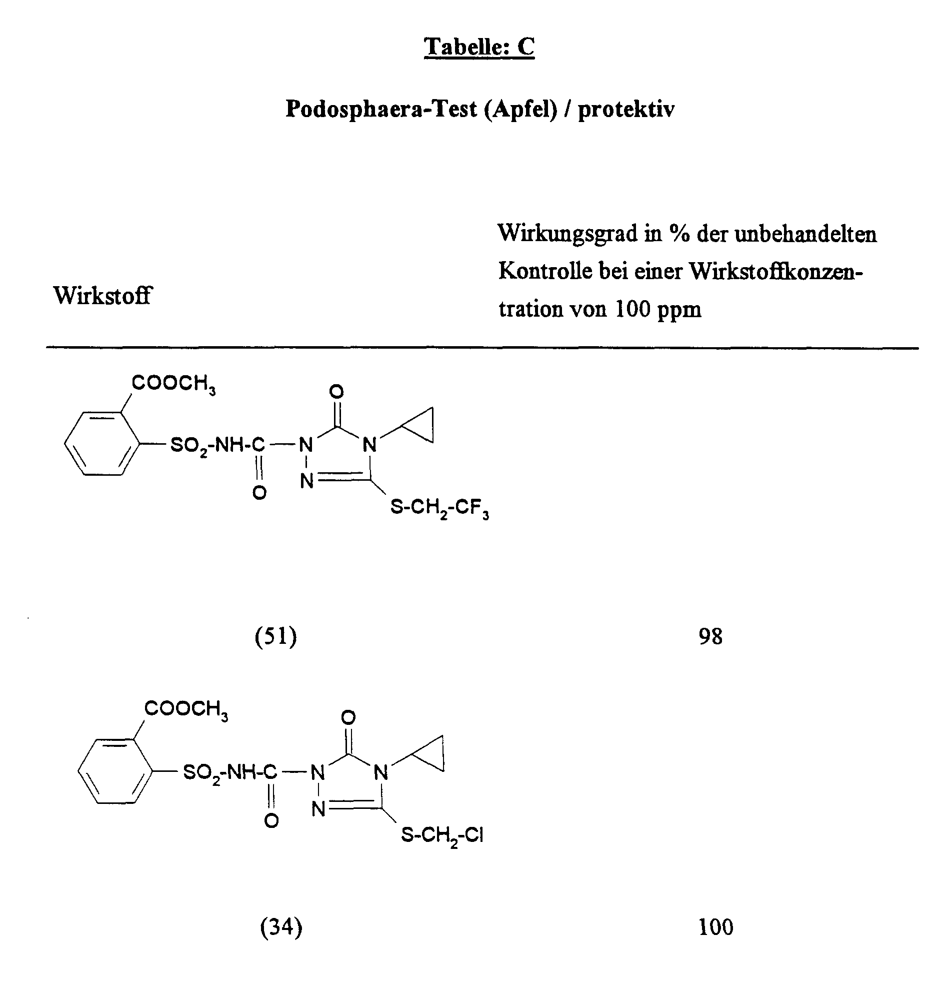

## Patentansprüche

1. Sulfonylaminocarbonyltriazolinone mit Halogenalkylthio-Substituenten der allgemeinen Formel (I), 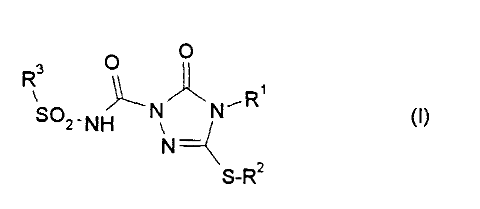 in welcher
R¹ für Wasserstoff, Hydroxy, Amino, Alkylidenamino oder für einen jeweils gegebenenfalls substituierten Rest aus der Reihe Alkyl, Alkenyl, Alkinyl, Alkylamino, Dialkylamino, Alkanoylamino, Cycloalkyl, Cycloalkylalkyl, Cycloalkylamino, Aryl, Arylalkyl steht,
R² für jeweils durch Halogen substituiertes Alkyl oder Alkenyl steht und
R³ für einen jeweils gegebenenfalls substituierten Rest aus der Reihe Alkyl, Aralkyl, Aryl, Heteroaryl steht,
sowie Salze von Verbindungen der Formel (I).

2. Verbindungen der Formel (I) gemäß Anspruch 1, dadurch gekennzeichnet, daß darin
R¹ für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor und/oder Chlor substituiertes C₁-C₆-Alkylamino, Di-(C₁-C₄-alkyl)-amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl oder Phenyl-C₁-C₄-alkyl steht,
R² für jeweils durch Fluor, Chlor und/oder Brom substituiertes C₁-C₆-Alkyl oder C₂-C₆-Alkenyl steht und
R³ für die Gruppierung 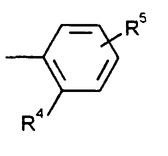 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxy-carbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl -carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxycarbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy- carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy- carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylaminocarbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkyl-sulfonyloxy, Di-(C₁-C₄-alkyl)-aminosulfonylamino oder für den Rest
-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl. Trifluormethoxy oder Trifluormethylthio substituiertes Phenyl. für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder fur gegebenenfalls durch Fluor, Chlor, Brom oder Methyl substituiertes Phenylsulfonylamino steht,
weiterhin
R³ für den Rest 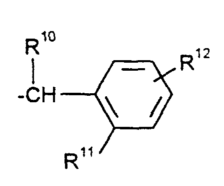 steht,
worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 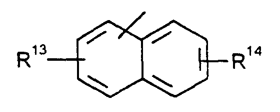 steht,
worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 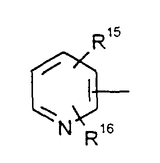 steht,
worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), für C₁-C₄-Alkylthio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
R³ für den Rest 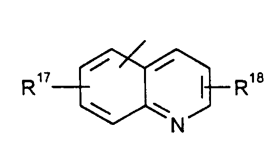 steht,
worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen;
weiterhin
R³ für den Rest 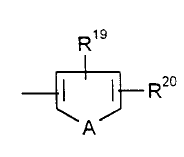 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 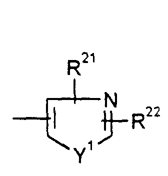 steht,
worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 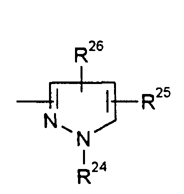 steht,
worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht,
sowie deren Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammo-nium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze.

3. Verbindungen der Formel (I) und deren Salze gemäß Anspruch 1, dadurch gekennzeichnet, daß darin
R¹ für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für Methylamino, Ethyl-amino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
R² für jeweils durch Fluor und/oder Chlor substituiertes Methyl, Ethyl, n-oder i-Propyl, n-, i-, s- oder t-Butyl, Propenyl oder Butenyl steht und
R³ für den Rest 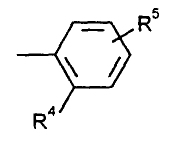 steht,
worin
R⁴ für Fluor, Chlor, Brom, Methyl, Ethyl, n- oder i-Propyl, Trifluormethyl, Methoxy, Ethoxy, n- oder i-Propoxy, n- oder i-Butoxy, Di-fluormethoxy, Trifluormethoxy, 2-Chlor-ethoxy, 2,2,2-Trifluorethoxy, 1,1,2,2-Tetrafluor-ethoxy, 1,1,2,2,2-Pentafluor-ethoxy, 2-Methoxy-ethoxy, Methylthio, Ethylthio, n- oder i-Propylthio, n-oder i-Butylthio, 2-Fluor-ethylthio, Allyloxy, Propargyloxy, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n-oder i-Propoxycarbonyl, steht und
R⁵ für Wasserstoff, Methyl, Ethyl, Fluor, Chlor oder Brom steht;
weiterhin
R³ für den Rest 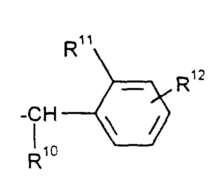 steht,
worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 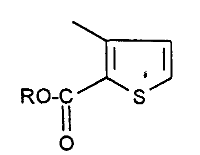 steht,
worin
R für Methyl, Ethyl, n- oder i-Propyl steht, oder
R³ für den Rest 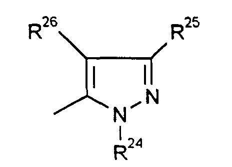 steht,
worin
R²⁴ für Methyl, Ethyl, n- oder i-Propyl, Phenyl oder Pyridyl steht,
R²⁵ für Wasserstoff, Fluor, Chlor oder Brom steht und
R²⁶ für Fluor, Chlor, Brom, Methoxycarbonyl oder Ethoxycarbonyl steht.

4. Verfahren zur Herstellung von Verbindungen der Formel (I) gemäß Anspruch 1 und deren Salzen, dadurch gekennzeichnet, daß man
(a) Triazolinone der allgemeinen Formel (II) 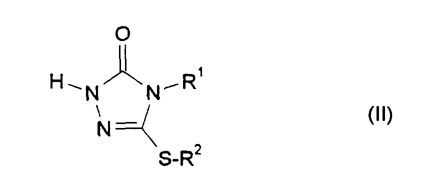 in welcher
R¹ und R² die in Anspruch 1 angegebene Bedeutung haben,
mit Sulfonylisocyanaten der allgemeinen Formel (III)
R³-SO₂-N=C=O (III)
in welcher
R³ die in Anspruch 1 angegebene Bedeutung hat,
gegebenenfalls in Gegenwart eines Reaktionshilfsmittels und gegebenenfalls in Gegenwart eines Verdünnungsmittels umsetzt,
oder daß man
(b) Triazolinon-Derivate der allgemeinen Formel (IV) 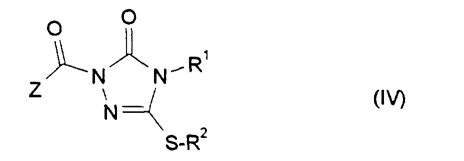 in welcher
R¹ und R² die oben angegebene Bedeutung haben und
Z für Halogen, Alkoxy, Aralkoxy oder Aryloxy steht,
mit Sulfonsäureamiden der allgemeinen Formel (V)
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
oder daß man
(c) Triazolinone der allgemeinen Formel (II) 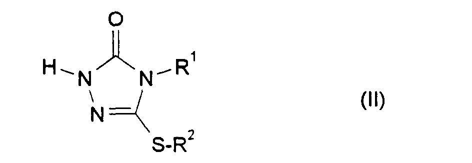 in welcher
R¹ und R² die oben angegebene Bedeutung haben,
mit Sulfonsäureamid-Derivaten der allgemeinen Formel (VI)
R³-SO₂-NH-CO-Z (VI)
in welcher
R³ die oben angegebene Bedeutung hat und
Z für Halogen, Alkoxy, Aralkoxy oder Aryloxy steht,
gegebenenfalls in Gegenwart eines Säureakzeptors und gegebenenfalls in Gegenwart eines Verdünnungsmittels umsetzt,
oder daß man
(d) Triazolinone der allgemeinen Formel (II) 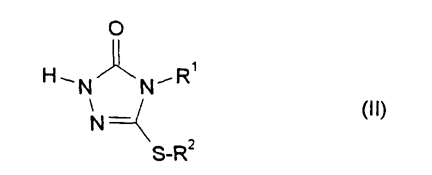 in welcher
R¹ und R² die oben angegebene Bedeutung haben,
mit Sulfonsäurehalogeniden der allgemeinen Formel (VII)
R³-SO₂-X (VII)
in welcher
R³ die oben angegebene Bedeutung hat und
X für Halogen steht,
und Metallcyanaten der allgemeinen Formel (VIII)
MOCN (VIII)
in welcher
M für ein Alkalimetall oder Erdalkalimetall-äquivalent steht,
gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt

5. Herbizide und/oder fungizide Mittel, gekennzeichnet durch einen Gehalt an mindestens einer Verbindung der Formel (I) gemäß Anspruch 1.

6. Verwendung von Verbindungen der allgemeinen Formel (I) gemäß Anspruch 1 zur Bekämpfung von unerwünschtem Pflanzenwachstum und/oder von phytopathogenen Pilzen.

7. Verfahren zur Bekämpfung von Unkräutern und/oder phytopathogenen Pilzen, dadurch gekennzeichnet, daß man Verbindungen der allgemeinen Formel (I) gemäß Anspruch 1 auf die Unkräuter bzw. die Pilze oder ihren Lebensraum einwirken läßt.

8. Verfahren zur Herstellung von herbiziden bzw. fungiziden Mitteln, dadurch gekennzeichnet, daß man Verbindungen der allgemeinen Formel (I) gemäß Anspruch 1 mit Streckmitteln und/oder oberflächenaktiven Mitteln vermischt.

9. Triazolinone der allgemeinen Formel (II) 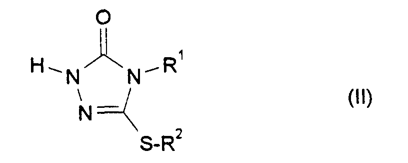 in welcher
R¹ für Wasserstoff, Hydroxy, Amino, Alkylidenamino oder für einen jeweils gegebenenfalls substituierten Rest aus der Reihe Alkyl, Alkenyl, Alkinyl Alkylamino, Dialkylamino, Alkanoylamino, Cycloalkyl, Cycloalkylalkyl. Cycloalkylamino, Aryl, Arylalkyl steht, und
R² für jeweils durch Halogen substituiertes Alkyl oder Alkenyl steht.

10. Triazolinon-Derivate der allgemeinen Formel (IV) 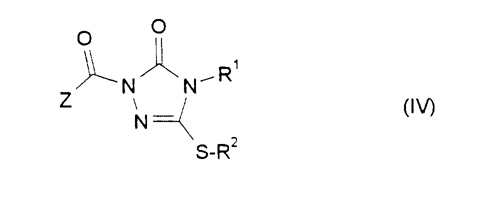 in welcher
R¹ für Wasserstoff, Hydroxy, Amino, Alkylidenamino oder für einen jeweils gegebenenfalls substituierten Rest aus der Reihe Alkyl, Alkenyl, Alkinyl, Alkylamino, Dialkylamino, Alkanoylamino, Cycloalkyl, Cycloalkylalkyl, Cycloalkylamino, Aryl, Arylalkyl steht,
R² für jeweils durch Halogen substituiertes Alkyl oder Alkenyl steht und
Z für Halogen, Alkoxy, Aralkoxy oder Aryloxy steht.

## Claims

1. Sulphonylaminocarbonyltriazolinones having halogenoalkylthio substituents of the general formula (I) 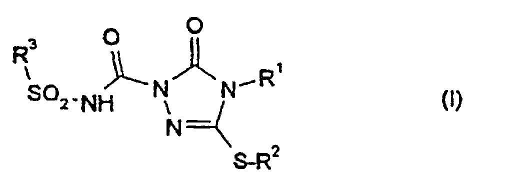 in which
R¹ represents hydrogen, hydroxyl, amino, alkylidenamino or a respectively optionally substituted radical from the group consisting of alkyl, alkenyl, alkinyl, alkylamino, dialkylamino, alkanoylamino, cycloalkyl, cycloalkylalkyl, cycloalkylamino, aryl, arylalkyl,
R² represents respectively halogen-substituted alkyl or alkenyl and
R³ represents a respectively optionally substituted radical from the group consisting of alkyl, aralkyl, aryl, heteroaryl,
and salts of compounds of the formula (I).

2. Compounds of the formula (I) according to Claim 1, characterized in that therein
R¹ represents hydrogen, hydroxyl, amino, C₁-C₆-alkylideneamino, or optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, or respectively optionally fluorine-, chlorine- and/or bromine-substituted C₂-C₆-alkenyl or C₂-C₆-alkinyl, or respectively optionally fluorine-and/or chlorine-substituted C₁-C₆-alkylamino, di-(C₁-C₄-alkyl)-amino or C₁-C₄-alkanoylamino, or respectively optionally fluorine-, chlorine-, bromine- and/or C₁-C₄-alkyl-substituted C₃-C₆-cycloalkyl or C₃-C₆-cycloalkyl-C₁-C₄-alkyl, or respectively optionally fluorine-, chlorine-, bromine-, cyano-, nitro-, C₁-C₄-alkyl-, trifluoromethyl-, C₁-C₄-alkoxy-and/or C₁-C₄-alkoxy-carbonyl-substituted phenyl or phenyl-C₁-C₄-alkyl,
R² represents respectively fluorine-, chlorine- and/or bromine-substituted C₁-C₆-alkyl or C₂-C₆-alkenyl and
R³ represents the grouping 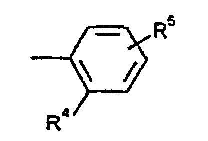
in which
R⁴ and R⁵ are identical or different and each represent hydrogen, fluorine, chlorine, bromine, iodine, nitro, C₁-C₆-alkyl, (which is optionally substituted by fluorine, chlorine, bromine, cyano, carboxyl, C₁-C₄-alkoxy-carbonyl, C₁-C₄-alkylamino-carbonyl, di-(C₁-C₄-alkyl)amino-carbonyl, hydroxyl, C₁-C₄-alkoxy, formyloxy, C₁-C₄-alkylcarbonyloxy, C₁-C₄-alkoxy-arbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), or C₂-C₆-alkenyl (which is optionally substituted by fluorine, chlorine, bromine, cyano, C₁-C₄-alkoxycarbonyl, carboxyl or phenyl), or C₂-C₆-alkinyl (which is optionally substituted by fluorine, chlorine, bromine, cyano, C₁-C₄-alkoxy-carbonyl, carboxyl or phenyl), or C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl), or C₂-C₆-alkenyloxy (which is optionally substituted by fluorine, chlorine, bromine, cyano or C₁-C₄-alkoxy-carbonyl), or C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or the radical -S(O)ₚ-R⁶, where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl or the radical -NHOR⁷, where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or benzohydryl or phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ further each represent phenyl or phenoxy, or C₁-C₄-alkyl-carbonylamino, C₁-C₄-alkoxy-arbonylamino, C₁-C₄-alkylamino-carbonyl-amino, di-(C₁-C₄-alkyl)-amino-carbonylamino, or the radical -CO-R⁸, where
R⁸ represents hydrogen, C₁-C₆-alkyl, C₃-C₆-cycloalkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (each of which are optionally substituted by fluorine and/or chlorine),
R⁴ and/or R⁵ further each represent trimethylsilyl, thiazolinyl, C₁-C₄-alkylsulphonyloxy, di-(C₁-C₄-alkyl)-aminosulphonyl-amino or the radical
-CH=N-R⁹, where
R⁹ represents optionally fluorine-, chlorine-, cyano-, carboxyl-, C₁-C₄-alkoxy-, C₁-C₄-alkylthio-, C₁-C₄-alkylsulphinyl- or C₁-C₄-alkylsulphonyl-substituted C₁-C₆-alkyl, or optionally fluorine- or chlorine-substituted benzyl, or optionally fluorine-or chlorine-substituted C₃-C₆-alkenyl or C₃-C₆-alkinyl, or optionally fluorine-, chlorine-, bromine-, C₁-C₄-alkyl-, C₁-C₄-alkoxy-, trifluoromethyl-, trifluoromethoxy- or trifluoromethylthio-substituted phenyl, or optionally fluorine-and/or chlorine-substituted C₁-C₆-alkoxy, C₃-C₆-alkenoxy, C₃-C₆-alkinoxy or benzyloxy, or amino, C₁-C₄-alkylamino, di-(C₁-C₄-alkyl)-amino, phenylamino, C₁-C₄-alkyl-carbonyl-amino, C₁-C₄-alkoxy-carbonylamino, C₁-C₄-alkyl-sulphonylamino or optionally fluorine-, chlorine-, bromine- or methyl-substituted phenylsulphonylamino,
furthermore
R³ represents the radical 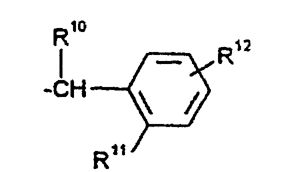
in which
R¹⁰ represents hydrogen or C₁-C₄-alkyl,
R¹¹ and R¹² are identical or different and each represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-aminosulphonyl;
furthermore
R³ represents the radical 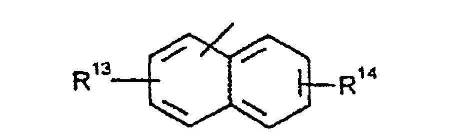 in which
R¹³ and R¹⁴ are identical or different and each represent hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 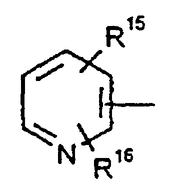
in which
R¹⁵ and R¹⁶ are identical or different and each represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), or C₁-C₄-alkylthio, C₁-C₄-alkylsulphinyl or C₁-C₄-alkylsulphonyl (which is optionally substituted by fluorine and/or chlorine), or aminosulphonyl, mono-(C₁-C₄-alkyl)-aminosulphonyl, or di-(C₁-C₄-alkyl)-aminosulphonyl or C₁-C₄-alkoxy-carbonyl or dimethylaminocarbonyl;
furthermore
R³ represents the radical 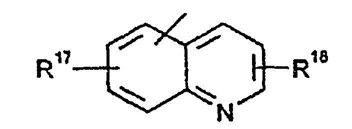 in which
R¹⁷ and R¹⁸ are identical or different and each represent hydrogen, fluorine, chlorine, bromine, C₁-C₄-alkyl (which is optionally substituted by fluorine and/or bromine), C₁-C₄-alkoxy (which is optionally substituted by fluorine and/or chlorine), or C₁-C₄-alkylthio, C₁-C₄-alkylsulphinyl or C₁-C₄-alkylsulphonyl (each of which are optionally substituted by fluorine and/or chlorine), or di-(C₁-C₄-alkyl)-aminosulphonyl;
furthermore
R³ represents the radical 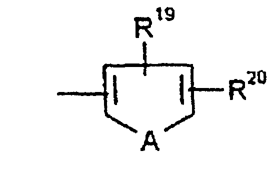 in which
R¹⁹ and R²⁰ are identical or different and each represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-amino-sulphonyl, C₁-C₄-alkoxy-carbonyl or dimethylaminocarbonyl, and
A represents oxygen, sulphur or the grouping N-Z¹, where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di-(C₁-C₄-alkyl)-aminocarbonyl;
furthermore
R³ represents the radical 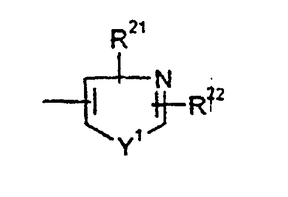 in which
R²¹ and R²² are identical or different and each represent hydrogen, C₁-C₄-alkyl, halogen, C₁-C₄-alkoxycarbonyl, C₁-C₄-alkoxy or C₁-C₄-halogenoalkoxy,
Y¹ represents sulphur or the grouping N-R²³, where
R²³ represents hydrogen or C₁-C₄-alkyl;
furthermore
R³ represents the radical
in which
R²⁴ represents hydrogen, C₁-C₄-alkyl, benzyl, pyridyl, quinolyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl and
R²⁶ represents hydrogen, halogen or C₁-C₄-alkyl,
and their sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts.

3. Components of the formula (I) and salts thereof according to Claim 1, characterized in that therein
R¹ represents respectively optionally fluorine-, chlorine-, cyano-, methoxy-or ethoxy-substituted methyl, ethyl, n- or i-propyl, n-, i-, s- or t-butyl, or respectively optionally fluorine-, chlorine- or bromine-substituted propenyl, butenyl, propinyl or butinyl, or methylamino, ethylamino, n- or i-propylamino, n-, i-, s- or t-butylamino, dimethylamino or diethylamino, or respectively optionally fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, or respectively optionally fluorine-, chlorine-, bromine-, cyano-, methyl-, trifluoromethyl- or methoxy-substituted benzyl or phenyl,
R² represents respectively fluorine- and/or chlorine-substituted methyl, ethyl, n- or i-propyl, n-, i-, s- or t-butyl, propenyl or butenyl and
R³ represents the radical 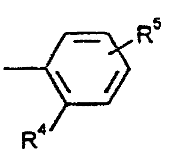 in which
R⁴ represents fluorine, chlorine, bromine, methyl, ethyl, n- or i-propyl, trifluoromethyl, methoxy, ethoxy, n- or i-propoxy, n- or i-butoxy, difluoromethoxy, trifluoromethoxy, 2-chloro-ethoxy, 2,2,2-trifluoro-ethoxy, 1,1,2,2-tetrafluoro-ethoxy, 1,1,2,2,2-pentafluoro-ethoxy, 2-methoxy-ethoxy, methylthio, ethylthio, n- or i-propylthio, n-or i-butylthio, 2-fluoro-ethylthio, allyloxy, propargyloxy, methylsulphinyl, ethylsulphinyl, methylsulphonyl, ethylsulphonyl, dimethylaminosulphonyl, diethylaminosulphonyl, N-methoxy-N-methylamino-sulphonyl, phenyl, phenoxy, methoxycarbonyl, ethoxycarbonyl, n- or i-propoxycarbonyl, and
R⁵ represents hydrogen, methyl, ethyl, fluorine, chlorine or bromine;
furthermore
R³ represents the radical 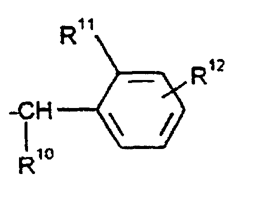 in which
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 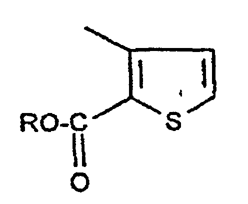 in which
R represents methyl, ethyl, n- or i-propyl, or
R³ represents the radical 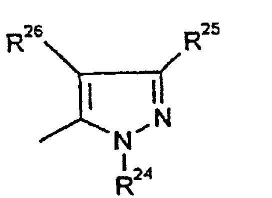
in which
R²⁴ represents methyl, ethyl, n- or i-propyl, phenyl or pyridyl,
R²⁵ represents hydrogen, fluorine, chlorine or bromine and
R²⁶ represents fluorine, chlorine, bromine, methoxycarbonyl or ethoxycarbonyl.

4. Process for preparing compounds of the formula (I) according to Claim 1 and salts thereof, characterized in that
(a) triazolinones of the general formula (II) 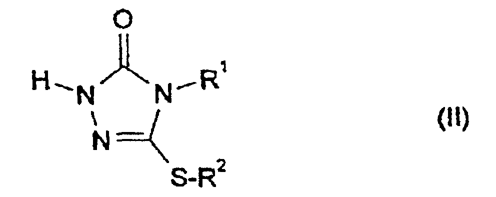 in which
R¹ and R² are each as defined in Claim 1
are reacted with sulphonyl isocyanates of the general formula (III)
R³-SO₂-N=C=O (III)
in which
R³ is as defined in Claim 1,
if appropriate in the presence of a reaction auxiliary and if appropriate in the presence of a diluent,
or
(b) triazolinone derivatives of the general formula (IV) 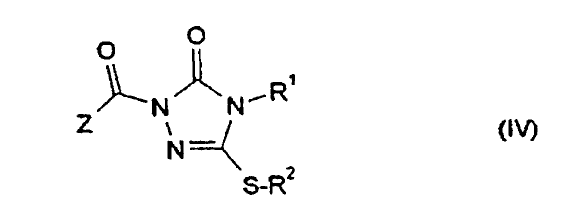 in which
R¹ and R² are each as defined above and
Z represents halogen, alkoxy, aralkoxy or aryloxy
are reacted with sulphonamides of the general formula (V)
R³-SO₂-NH₂ (V)
in which
R³ is as defined above,
if appropriate in the presence of an acid acceptor and if appropriate in the presence of a diluent, or
(c) triazolinones of the general formula (II) 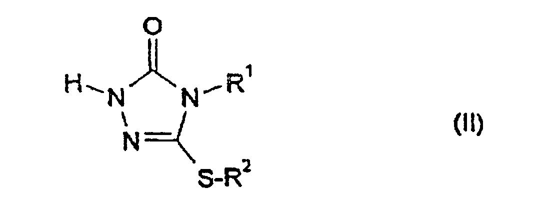 in which
R¹ and R² are each as defined above
are reacted with sulphonamide derivatives of the general formula (VI)
R³-SO₂-NH-CO-Z (VI)
in which
R³ is as defined above and
Z represents halogen, alkoxy, aralkoxy or aryloxy,
if appropriate in the presence of an acid acceptor and if appropriate in the presence of a diluent,
or
(d) triazolinones of the general formula (II) 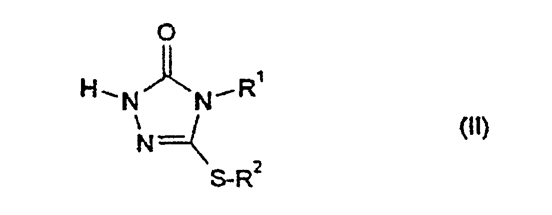 in which
R¹ and R² are each as defined above
are reacted with sulphonyl halides of the general formula (VII)
R³-SO₂-X (VII)
in which
R³ is as defined above and
X represents halogen
and metal cyanates of the general formula (VIII)
MOCN (VIII)
in which
M represents an alkali metal or an alkaline earth metal equivalent,
if appropriate in the presence of a reaction auxiliary and if appropriate in the presence of a diluent,
and the compounds of the formula (I) obtained by process (a), (b), (c) or (d) are, if required, converted by customary methods into salts.

5. Herbicidal and/or fungicidal compositions, characterized by a content of at least one compound of the formula (I) according to Claim 1.

6. Use of compounds of the general formula (I) according to Claim 1 for controlling undesirable vegetation and/or phytopathogenic fungi.

7. Method for controlling weeds and/or phytopathogenic fungi, characterized in that compounds of the general formula (I) according to Claim 1 are allowed to act on the weeds or the fungi or their habitat.

8. Process for preparing herbicidal and/or fungicidal compositions, characterized in that compounds of the general formula (I) according to Claim 1 are mixed with extenders and/or surface-active agents.

9. Triazolinones of the general formula (II) 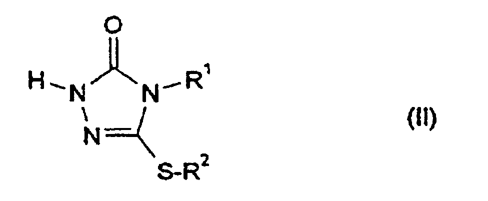 in which
R¹ represents hydrogen, hydroxyl, amino, alkylideneamino or a respectively optionally substituted radical from the group consisting of alkyl, alkenyl, alkinyl, alkylamino, dialkylamino, alkanoylamino, cycloalkyl, cycloalkylalkyl, cycloalkylamino, aryl, arylalkyl, and
R² represents respectively halogen-substituted alkyl or alkenyl.

10. Triazolinone derivatives of the general formula (IV) 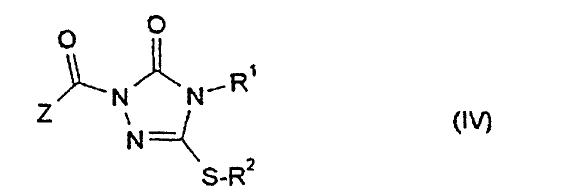 in which
R¹ represents hydrogen, hydroxyl, amino, alkylideneamino or a respectively optionally substituted radical from the group consisting of alkyl, alkenyl, alkinyl, alkylamino, dialkylamino, alkanoylamino, cycloalkyl, cycloalkylalkyl, cycloalkylamino, aryl, arylalkyl,
R² represents respectively halogen-substituted alkyl or alkenyl and
Z represents halogen, alkoxy, aralkoxy, or aryloxy.

## Revendications

1. Sulfonylaminocarbonyltriazolinones à substituants halogénalkylthio, de formule générale (I) 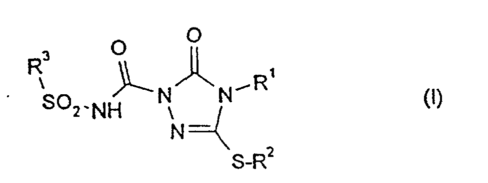 dans laquelle
R¹ représente l'hydrogène, un groupe hydroxy, amino, alkylidène-amino ou un reste, éventuellement substitué dans chaque cas, de la série alkyle, alcényle, alcynyle, alkylamino, dialkylamino, alcanoylamino, cycloalkyle, cycloalkylalkyle, cycloalkylamino, aryle, arylalkyle,
R² représente un groupe alkyle ou alcényle substitués chacun par un halogène et
R³ représente un reste, éventuellement substitué dans chaque cas, de la série alkyle, aralkyle, aryle, hétéroaryle,
ainsi que des sels de composés de formule (I)

2. Composés de formule (I) suivant la revendication 1, caractérisés en ce que, dans la formule,
R¹ représente l'hydrogène, un groupe hydroxy, amino, (alkylidène en C₁ à C₆) amino, un groupe alkyle en C₁ à C₆ éventuellement substitué par un radical fluoro, chloro, bromo, cyano, alkoxy en C₁ à C₄, (alkyle en C₁ à C₄)-carbonyle ou (alkoxy en C₁ à C₄)-carbonyle, un groupe alcényle en C₂ à C₆ ou un groupe alcynyle en C₂ à C₆ substitués chacun le cas échéant par du fluor, du chlore et/ou du brome, un groupe alkylamino en C₁ à C₆, un groupe di-(alkyle en C₁ à C₄) -amino ou un groupe alcanoylamino en C₁ à C₄ substitués chacun le cas échéant par du fluor et/ou du chlore, un groupe cycloalkyle en C₃ à C₆ ou un groupe (cycloalkyle en C₃ à C₆)-(alkyle en C₁ à C₄) substitués chacun le cas échéant par un radical fluoro, chloro, bromo et/ou alkyle en C₁ à C₄, ou bien un groupe phényle ou un groupe phényl-(alkyle en C₁ à C₄) substitués chacun le cas échéant par un radical fluoro, chloro, bromo, cyano, nitro, alkyle en C₁ à C₄, trifluorométhyle, alkoxy en C₁ à C₄ et/ou (alkoxy en C₁ à C₄)-carbonyle,
R² représente un groupe alkyle en C₁ à C₆ ou un groupe alcényle en C₂ à C₆ substitués chacun par du fluor, du chlore et/ou du brome et
R³ représente le groupement 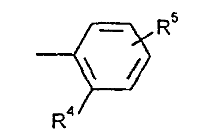 dans lequel
R⁴ et R⁵ sont identiques ou différents et représentent l'hydrogène, le fluor, le chlore, le brome, l'iode, un groupe nitro, alkyle en C₁ à C₆ (qui est éventuellement substitué par du fluor, du chlore, du brome, un radical cyano, carboxy, (alkoxy en C₁ à C₄)-carbonyle, (alkylamino en C₁ à C₄)-carbonyle, di-(alkyle en C₁ à C₄) aminocarbonyle, hydroxy, alkoxy en C₁ à C₄, formyloxy, (alkyle en C₁ à C₄)-carbonyloxy, (alkoxy en C₁ à C₄)-carbonyloxy, (alkylamino en C₁ à C₄)-carbonyloxy, alkylthio en C₁ à C₄, alkylsulfinyle en C₁ à C₄, alkylsulfonyle en C₁ à C₄, di-(alkyle en C₁ à C₄) aminosulfonyle, cycloalkyle en C₃ à C₆ ou phényle), un groupe alcényle en C₂ à C₆ (qui est éventuellement substitué par un radical fluoro, chloro, bromo, cyano, (alkoxy en C₁ à C₄)-carbonyle, carboxy ou phényle), un groupe alcynyle en C₂ à C₆ (qui est éventuellement substitué par un radical fluoro, chloro, bromo, cyano, (alkoxy en C₁ à C₄)-carbonyle, carboxy ou phényle), un groupe alkoxy en C₁ à C₄ (qui est substitué le cas échéant par un radical fluoro, chloro, bromo, cyano, carboxy (alkoxy en C₁ à C₄)-carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est substitué le cas échéant par un radical fluoro, chloro, bromo, cyano, carboxy, (alkoxy en C₁ à C₄)-carbonyle, alkylthio en C₁ à C₄, alkylsulfinyle en C₁ à C₄ ou alkylsulfonyle en C₁ à C₄), un groupe alcényloxy en C₂ à C₆ (qui est substitué la cas échéant par un radical fluoro, chloro, bromo, cyano ou (alkoxy en C₁ à C₄) - carbonyle), un groupe alcénylthio en C₂ à C₆ (qui est substitué le cas échéant par un radical fluoro, chloro, bromo, cyano, nitro, alkylthio ou (alkoxy en C₁ à C₄)-carbonyle), un groupe alcynyloxy en C₃ à C₆, alcynylthio en C₃ à C₆ ou le reste -S(O)ₚ-R⁶ dans lequel
p représente les nombres 1 ou 2 et
R⁶ est un groupe alkyle en C₁ à C₄ (qui est substitué le cas échéant par un radical fluoro, chloro, bromo, cyano ou (alkoxy en C₁ à C₄)-carbonyle), un groupe alcényle en C₃ à C₆, alcynyle en C₃ à C₆, alkoxy en C₁ à C₄, (alkoxy en C₁ à C₄) - (alkylamino en C₁ à C₄), alkylamino en Ci à C₄, di-(alkyle en C₁ à C₄)amino, phényle ou le reste -NHOR⁷ dans lequel
R⁷ représente un groupe alkyle en C₁ à C₁₂ (qui est substitué le cas échéant par un radical fluoro, chloro, cyano, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle, (alkoxy en C₁ à C₄)-carbonyle, (alkylamino en C₁ à C₄)-carbonyle, ou di-(alkyle en C₁ à C₄)-aminocarbonyle), un groupe alcényle en C₃ à C₆ (qui est substitué le cas échéant par du fluor, du chlore ou du brome), un groupe alcynyle en C₃ à C₆, cycloalkyle en C₃ à C₆, (cycloalkyle en C₃ à C₆-(alkyle en C₁ ou C₂), phényl-(alkyle en C₁ ou C₂) (qui est substitué le cas échéant par un radical fluoro, chloro, nitro, cyano, alkyle en C₁ à C₄, alkoxy en C₁ à C₄ ou (alkoxy en C₁ à C₄)-carbonyle), un groupe benzhydryle ou un groupe phényle (qui est substitué le cas échéant par un radical fluoro, chloro, nitro, cyano, alkyle en C₁ à C₄, trifluorométhyle, alkoxy en C₁ à C₄, fluoralkoxy en C₁ ou C₂, alkylthio en C₁ à C₄, trifluorométhylthio ou (alkoxy en C₁ à C₄) -carbonyle),
R⁴ et/ou R⁵ représentent en outre un groupe phényle ou phénoxy, un groupe (alkyle en C₁ à C₄)-carbonylamino, (alkoxy en C₁ à C₄)-carbonylamino, (alkylamino en C₁ à C₄)-carbonylamino, di-(alkyle en C₁ à C₄)aminocarbonylamino ou le reste -CO-R⁸ dans lequel
R⁸ est l'hydrogène, un groupe alkyle en C₁ à C₆, cycloalkyle en C₃ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-alkylamino en C₁ à C₄ ou di-(alkyle en C₁ à C₄)-amino (qui sont éventuellement substitués par du fluor et/ou du chlore),
R⁴ et/ou R⁵ représentent en outre un reste triméthylsilyle, thiazolinyle, alkylsulfonyloxy en C₁ à C₄, di-(alkyle en C₁ à C₄)aminosulfonylamino ou le reste
-CH=N-R⁹ dans lequel
R⁹ représente un groupe alkyle en C₁ à C₆ éventuellement substitué par un radical fluoro, chloro, cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou un groupe alcynyle en C₃ à C₆ éventuellement substitués par du fluor ou du chlore, un groupe phényle éventuellement substitué par un radical fluoro, chloro, bromo, alkyle en C₁ à C₄, alkoxy en C₁ à C₄, trifluorométhyle, trifluorométhoxy ou trifluorométhylthio, un groupe benzyloxy, alcynyloxy en C₃ à C₆, alcényloxy en C₃ à C₆ ou alkoxy en C₁ à C₆ portant éventuellement un substituant fluoro et/ou chloro, un groupe amino, alkylamino en C₁ à C₄, di-(alkyle en C₁ à C₄)amino, phénylamino, (alkyle en C₁ à C₄)carbonylamino, (alkoxy en C₁ à C₄)-carbonylamino, alkylsulfonylamino en C₁ à C₄ ou un groupe phénylsulfonylamino éventuellement substitué par un radical fluoro, chloro, bromo ou méthyle,
en outre
R³ représente le reste 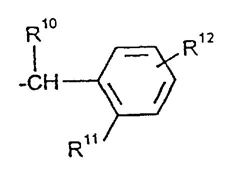 dans lequel
R¹⁰ est l'hydrogène ou un groupe alkyle en C₁ à C₄,
R¹¹ et R¹² sont identiques ou différents et représentent l'hydrogène, le fluor, le chlore, le brome, un groupe nitro, cyano, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe carboxy, (alkoxy en C₁ à C₄) carbonyle, diméthylaminocarbonyle, alkylsulfonyle en C₁ à C₄ ou di-(alkyle en C₁ à C₄) aminosulfonyle ;
en outre
R³ représente le reste 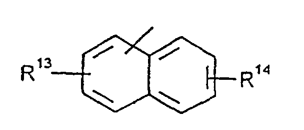 dans lequel
R¹³ et R¹⁴ sont identiques ou différents et représentent l'hydrogène, le fluor, le chlore, le brome, un groupe nitro, cyano, alkyle en C₁ à C₄ (qui est substitué le cas échéant par du fluor et/ou du chlore) ou un groupe alkoxy en C₁ à C₄ (qui est substitué le cas échéant par du fluor et/ou du chlore) ;
en outre
R³ représente le reste 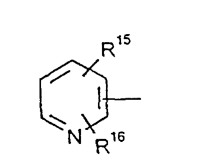 dans lequel
R¹⁵ et R¹⁶ sont identiques ou différents et représentent l'hydrogène, le fluor, le chlore, le brome, un groupe nitro, cyano, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe alkylthio en C₁ à C₄, un groupe alkylsulfinyle en C₁ à C₄ ou un groupe alkylsulfonyle en C₁ à C₄ (qui sont substitués le cas échéant par du fluor et/ou du chlore), un groupe aminosulfonyle, mono(alkyle en C₁ à C₄)-aminosulfonyle, di- (alkyle en C₁ à C₄) - aminosulfonyle ou (alkoxy en C₁ à C₄)-carbonyle ou un groupe diméthylaminocarbonyle ;
en outre
R³ représente le reste 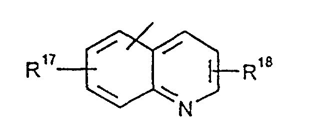 dans lequel
R¹⁷ et R¹⁸ sont identiques ou différents et représentent l'hydrogène, le fluor, le chlore, le brome, un groupe alkyle en C₁ à C₄ (qui est substitué le cas échéant par du fluor et/ou du brome), un groupe alkoxy en C₁ à C₄ (qui est substitué le cas échéant par du fluor et/ou du chlore), un groupe alkylthio en C₁ à C₄, un groupe alkylsulfinyle en C₁ à C₄ ou un groupe alkylsulfonyle en C₁ à C₄ (qui sont substitués le cas échéant par du fluor et/ou du chlore) ou un groupe di-(alkyle en C₁ à C₄)-aminosulfonyle ;
en outre
R³ représente le reste 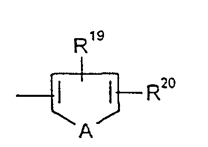 dans lequel
R¹⁹ et R²⁰ sont identiques ou différents et représentent l'hydrogène, le fluor, le chlore, le brome, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe alkylthio en C₁ à C₄, un groupe alkylsulfinyle en C₁ à C₄ ou un groupe alkylsulfonyle en C₁ à C₄ (qui sont substitués le cas échéant par du fluor et/ou du chlore), un groupe di-(alkyle en C₁ à C₄) aminosulfonyle, (alkoxy en C₁ à C₄)-carbonyle ou diméthylaminocarbonyle, et
A représente l'oxygène, le soufre ou le groupement N-Z¹ dans lequel
Z¹ est l'hydrogène, un groupe alkyle en C₁ à C₄ (qui est substitué le cas échéant par un radical fluoro, chloro, bromo ou cyano), un groupe cycloalkyle en C₃ à C₆, benzyle, un groupe phényle (qui est substitué le cas échéant par un radical fluoro, chloro, bromo ou nitro), un groupe (alkyle en C₁ à C₄)-carbonyle, (alkoxy en C₁ à C₄)-carbonyle ou di-(alkyle en C₁ à C₄)-aminocarbonyle ; en outre
R³ représente le reste 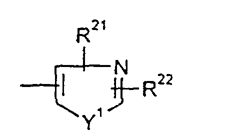 dans lequel
R²¹ et R²² sont identiques ou différents et représentent l'hydrogène, un groupe alkyle en C₁ à C₄, un halogène, un groupe (alkoxy en C₁ à C₄) - carbonyle, alkoxy en C₁ à C₄ ou halogénalkoxy en C₁ à C₄,
Y¹ représente le soufre ou le groupement N-R²³ dans lequel
R²³ est l'hydrogène ou un groupe alkyle en C₁ à C₄ ; en outre
R³ représente le reste 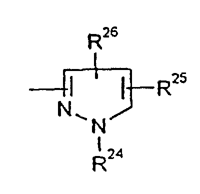 dans lequel
R²⁴ représente l'hydrogène, un groupe alkyle en C₁ à C₄, benzyle, pyridyle, quinolinyle ou phényle,
R²⁵ représente l'hydrogène, un halogène, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe dioxolanyle ou (alkoxy en C₁ à C₄)-carbonyle et
R²⁶ représente l'hydrogène, un halogène ou un groupe alkyle en C₁ à C₄,
ainsi que leurs sels de sodium, de potassium, de magnésium, de calcium, d'ammonium, de (alkyle en C₁ à C₄)-ammonium, di-(alkyle en C₁ à C₄)-ammonium, de tri-(alkyle en C₁ à C₄)-ammonium, de tétra-(alkyle en C₁ à C₄)-ammonium, de tri-(alkyle en C₁ à C₄)-sulfonium, de (cycloalkyle en C₅ ou C₆)-ammonium et de di(alkyle en C₁ ou C₂)-benzylammonium.

3. Composés de formule (I) et leurs sels suivant la revendication 1, caractérisé en ce que, dans la formule,
R¹ représente un groupe méthyle, éthyle, n-propyle, isopropyle, n-butyle, isobutyle, sec.-butyle, tertiobutyle portant éventuellement dans chaque cas un substituant fluoro, chloro, cyano, méthoxy ou éthoxy, un groupe propényle, butényle, propynyle ou butynyle portant éventuellement dans chaque cas un substituant fluoro, chloro ou bromo, un groupe méthylamino, éthylamino, n-propylamino, isopropylamino, n-butylamino, isobutylamino, sec.-butylamino, tertiobutylamino, diméthylamino ou diéthylamino, un groupe cyclopropyle, cyclobutyle, cyclopentyle, cyclohexyle, cyclopropylméthyle, cyclobutylméthyle, cyclopentylméthyle ou cyclohexylméthyle portant éventuellement dans chaque cas un substituant fluoro, chloro, bromo, méthyle ou éthyle, ou bien un groupe benzyle ou phényle portant éventuellement dans chaque cas un substituant fluoro, chloro, bromo, cyano, méthyle, trifluorométhyle ou méthoxy,
R² représente un groupe méthyle, éthyle, n-propyle, isopropyle, n-butyle, isobutyle, sec.-butyle, tertiobutyle, propényle ou butényle portant dans chaque cas un substituant fluoro et/ou chloro et
R³ représente le reste 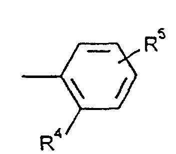 dans lequel
R⁴ représente le fluor, le chlore, le brome, un groupe méthyle, éthyle, n-propyle, isopropyle, trifluorométhyle, méthoxy, éthoxy, n-propoxy, isopropoxy, n-butoxy, isobutoxy, difluorométhoxy, trifluorométhoxy, 2-chloréthoxy, 2,2,2-trifluoréthoxy, 1,1,2,2-tétrafluoréthoxy, 1,1,2,2,2-pentafluoréthoxy, 2-méthoxyéthoxy, méthylthio, éthylthio, n-propylthio, isopropylthio, n-butyl-thio, isobutylthio, 2-fluoréthylthio, allyloxy, propargyloxy, méthylsulfinyle, éthylsulfinyle, méthylsulfonyle, éthylsulfonyle, diméthylaminosulfonyle, diéthylaminosulfonyle, N-méthoxy-N-méthylaminosulfonyle, phényle, phénoxy, méthoxycarbonyle, éthoxycarbonyle, n-propoxycarbonyle ou isopropoxycarbonyle et
R⁵ représente l'hydrogène, un groupe méthyle, éthyle, le fluor, le chlore ou le brome ;
en outre
R³ représente le reste 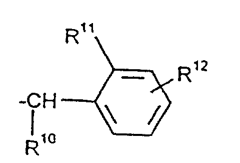 dans lequel
R¹⁰ est l'hydrogène
R¹¹ représente le fluor, le chlore, le brome, un groupe méthyle, méthoxy, difluorométhoxy, trifluorométhoxy, éthoxy, méthoxycarbonyle, éthoxycarbonyle, méthylsulfonyle ou diméthylamino-sulfonyle et
R¹² est l'hydrogène ;
en outre
R³ représente le reste 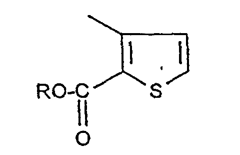 dans lequel
R est un groupe méthyle, éthyle, n-propyle ou isopropyle, ou bien
R³ représente le reste 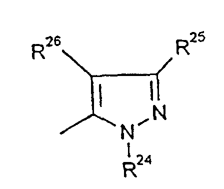 dans lequel
R²⁴ est un groupe méthyle, éthyle, n-propyle, isopropyle, phényle ou pyridyle,
R²⁵ est l'hydrogène, le fluor, le chlore ou le brome et
R²⁶ est le fluor, le chlore, le brome, un groupe méthoxycarbonyle ou éthoxycarbonyle.

4. Procédé de production de composés de formule (I) suivant la revendication 1 et de leurs sels, caractérisé en ce que
(a) on fait réagir des triazolinones de formule générale (II) 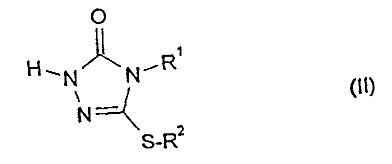 dans laquelle R¹ et R² ont la définition indiquée dans la revendication 1
avec des sulfonylisocyanates de formule générale (III)
R³-SO₂-N=C=O (III)
dans laquelle
R³ a la définition indiquée dans la revendication 1 le cas échéant en présence d'un auxiliaire de réaction et en présence éventuelle d'un diluant, ou bien
(b) on fait réagir des dérivés de triazolinone de formule générale (IV) 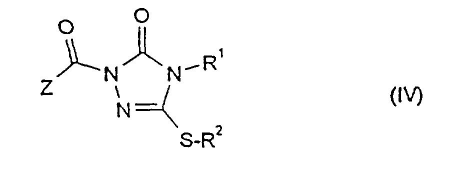 dans laquelle
R¹ et R² ont la définition indiquée ci-dessus et
Z représente un halogène, un groupe alkoxy, aralkoxy ou aryloxy
avec des amides d'acide sulfonique de formule générale (V)
R³-SO₂-NH₂ (V)
dans laquelle
R³ a la définition indiquée ci-dessus,
le cas échéant en présence d'un accepteur d'acide et en présence éventuelle d'un diluant,
ou bien
(c) on fait réagir des triazolinones de formule générale (II) 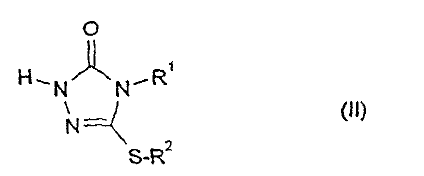 dans laquelle
R¹ et R² ont la définition indiquée ci-dessus
avec des dérivés d'amides d'acide sulfonique de formule générale (VI)
R³-SO₂-NH-CO-Z (VI)
dans laquelle
R³ a la définition indiquée ci-dessus et
Z est un halogène, un groupe alkoxy, aralkoxy ou aryloxy,
le cas échéant en présence d'un accepteur d'acide et en présence éventuelle d'un diluant,
ou bien
(d) on fait réagir des triazolinones de formule générale (II) 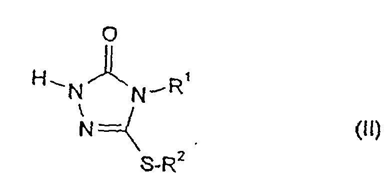 dans laquelle
R¹ et R² ont la définition indiquée ci-dessus,
avec des halogénures d'acide sulfonique de formule générale (VII)
R³-SO₂-X (VII)
dans laquelle
R³ a la définition indiquée ci-dessus set
X représente un halogène,
et des cyanates métalliques de formule générale (VIII)
MOCN (VIII)
dans laquelle
M représente un métal alcalin ou un équivalent de métal alcalino-terreux
le cas échéant en présence d'un auxiliaire de réaction et en présence éventuelle d'un diluant,
et on transforme éventuellement en sels par des procédés classiques les composés de formule (I) obtenus selon les procédés (a), (b), (c) ou (d).

5. Compositions herbicides et/ou fongicides, caractérisées par une teneur en au moins un composé de formule (I) suivant la revendication 1.

6. Utilisation de composés de formule générale
(I) suivant la revendication 1 pour combattre la croissance de plantes indésirables et/ou de champignons phytopathogènes.

7. Procédé pour combattre des mauvaises herbes et/ou des champignons phytopathogènes, caractérisé en ce qu'on fait agir des composés de formule générale (I) suivant la revendication 1 sur les mauvaises herbes ou les champignons ou sur leur milieu.

8. Procédé de préparation de compositions herbicides et/ou fongicides, caractérisé en ce qu'on mélange des composés de formule générale (I) suivant la revendication 1 avec des diluants et/ou des agents tensioactifs.

9. Triazolinones de formule générale (II) 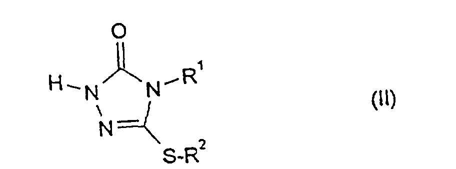 dans laquelle
R¹ représente l'hydrogène, un groupe hydroxy, amino, alkylidène-amino ou un reste, éventuellement substitué dans chaque cas, de la série alkyle, alcényle, alcynyle, alkylamino, dialkylamino, alcanoylamino, cycloalkyle, cycloalkylalkyle, cycloalkylamino, aryle, arylalkyle, et
R² est un groupe alkyle ou un groupe alcényle substitués chacun par un halogène.

10. Dérivés de triazolinone de formule générale (IV) 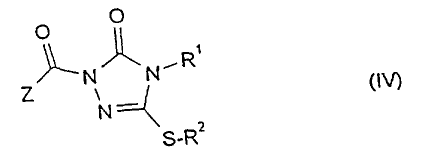 dans laquelle
R¹ représente l'hydrogène, un groupe hydroxy, amino, alkylidène-amino ou un reste, éventuellement substitué dans chaque cas, de la série alkyle, alcényle, alcynyle, alkylamino, dialkylamino, alcanoylamino, cycloalkyle, cycloalkylalkyle, cycloalkylamino, aryle, arylalkyle,
R² est un groupe alkyle ou un groupe alcényle substitués chacun par un halogène, et
Z est un halogène, un groupe alkoxy, aralkoxy ou aryloxy.
